# EUROPEAN PATENT SPECIFICATION

(11) **EP 4 066 823 B1**
(45) Date of publication and mention of the grant of the patent: **30.04.2025**
(21) Application number: 21165778.8
(22) Date of filing: 30.03.2021
(51) Int. Cl.: A61K 31/00, A61K 31/436, A61K 31/4439, A61K 31/517, A61K 31/519, A61K 31/5377, A61K 31/7004, A61P 35/00, G01N 33/50

(54) **REDUCED CALORIC INTAKE AND ANTICANCER AGENTS FOR THE TREATMENT OF CANCER**
REDUZIERTE KALORIENAUFNAHME UND ANTIKREBSMITTEL ZUR BEHANDLUNG VON KREBS
APPORT CALORIQUE RÉDUIT ET AGENTS ANTICANCÉREUX POUR LE TRAITEMENT DU CANCER

(43) Date of publication of application: 05.10.2022
(73) Proprietor: IFOM - Istituto Fondazione di Oncologia Molecolare ETS, 20139 Milano (IT)
(72) Inventor: LONGO, Valter, 16146 Genova (IT); SALVADORI, Giulia, 20158 Milano (IT)
(74) Representative: Pace Napoleone, Maria

(56) References cited:
- WO-A1-2020/157048
- WO-A2-2011/050302
- WO-A2-2015/134837
- DE GROOT STEFANIE ET AL: "Fasting mimicking diet as an adjunct to neoadjuvant chemotherapy for breast cancer in the multicentre randomized phase 2 DIRECT trial", NATURE COMMUNICATIONS, vol. 11, no. 1, 1 December 2020 (2020-12-01), XP055835240, Retrieved from the Internet <URL:https://www.ncbi.nlm.nih.gov/pmc/articles/PMC7311547/pdf/41467_2020_Article_16138.pdf> DOI: 10.1038/s41467-020-16138-3
- DELIGIORGI MARIA V. ET AL: "How Far Are We from Prescribing Fasting as Anticancer Medicine?", INTERNATIONAL JOURNAL OF MOLECULAR SCIENCES, vol. 21, no. 23, 1 December 2020 (2020-12-01), pages 9175, XP055835415, DOI: 10.3390/ijms21239175
- SONG MENGQIU ET AL: "AKT as a Therapeutic Target for Cancer", CANCER RESEARCH, vol. 79, no. 6, 15 March 2019 (2019-03-15), pages 1019 - 1031, XP055835371, ISSN: 0008-5472, Retrieved from the Internet <URL:https://cancerres.aacrjournals.org/content/canres/early/2019/02/25/0008-5472.CAN-18-2738.full.pdf> DOI: 10.1158/0008-5472.CAN-18-2738
- SALVADORI GIULIA ET AL: "Fasting-mimicking diet blocks triple-negative breast cancer and cancer stem cell escape", CELL METABOLISM, CELL PRESS, UNITED STATES, vol. 33, no. 11, 2 November 2021 (2021-11-02), pages 2247, XP086847790, ISSN: 1550-4131, [retrieved on 20211102], DOI: 10.1016/J.CMET.2021.10.008

## Description

### FIELD OF THE INVENTION

The present invention relates to the use of fasting mimicking diet (FMD) to inhibit growth and/or kill cancer stem cells (CSCs)in combination with at least one anticancer agent wherein the anticancer agent is an AKT inhibitor.

FMD in combination with said anticancer agent is particularly efficient in the treatment of triple negative breast cancer (TNBC) and reduces cancer stem cells (CSCs) escape pathways. The present disclosure further relates to a method to identify starvation escape pathways in differentiated cancer cells, which can be targeted by drugs.

### BACKGROUND OF THE INVENTION

Triple negative breast cancer (TNBC), the most aggressive breast cancer (BC) subtype, is characterized by high clinical aggressiveness and high recurrence rates when compared to other BC subtypes¹. Recently approved chemotherapy-immunotherapy combinations for the treatment of both limited-stage (in the neoadjuvant setting) and metastatic TNBC have improved patient prognosis in all disease settings²⁻⁴. However, the prognosis of the vast majority of advanced TNBC patients remains poor because of primary/acquired tumor resistance to treatments and of the presence of cancer stem cells (CSCs), which are responsible for tumor re-population after initial regressions. CSCs are a subset of slow cycling cancer cells characterized by low levels of intracellular reactive oxygen species (ROS), which contribute to their self-renewal potential and chemotherapy-radiotherapy resistance. CSCs can arise and survive under hypoxic conditions as a result of hypoxia inducible factor (HIF-1)-mediated upregulation of the expression of glucose transporters (GLUT) and glycolysis enzymes, such as hexokinase (HK)^{6,7}. This metabolic plasticity allows CSCs to survive in an unfavorable tumor microenvironment, thus making them the most resistant subset of cancer cells.

In recent years, it has been shown that cycles of fasting/fasting mimicking diet (FMD), based on severe calorie restriction of 50% or more, low levels of protein and sugars, and relatively high fat content enhance the efficacy of standard therapies on different types of cancer, including BC, while inducing the protection of normal cells from toxic side effects⁸⁻¹⁵. Fasting can induce these phenomena partly by reducing circulating insulin growth factor 1 (IGF-1), as well as glucose, leptin and insulin levels and by differentially regulating, in normal and cancer cells, genes involved in cellular protection, including antioxidant activity and DNA repair. Previous fasting/FMD-based interventions against TNBC, which involved chemotherapy, slowed tumor progression but did not achieve long-term progression free survival and reduced, but did not completely prevent, the damage to normal cells and systems^{8,10}. Because TNBC progression is reported to be dependent on CSCs, and due to the key role of CSCs in tumor initiation, progression and drug resistance, it is crucial to understand the effect of FMD on CSCs survival and how this may affect TNBC survival and growth.

Metastatic tumors remain lethal due to primary/acquired resistance to therapy or cancer stem cell-mediated repopulation. In the present invention it has been found that a fasting-mimicking diet (FMD) activates starvation escape pathways in triple negative breast cancer cells, which can be identified and targeted by drugs. In cancer stem cells, FMD cycles lower glucose-dependent protein kinase A signaling and stemness markers to reduce cell number and increase mouse survival. Accordingly, metastatic triple negative breast cancer patients with lower glycemia survive longer than those with higher baseline glycemia. By contrast, in differentiated cancer cells, FMD cycles activate the PI3K-AKT, mTOR, and CDK4/6 pathways, targeted by drugs to promote tumor regression. FMD cycles also prevent the hyperglycemia and toxicity caused by these drugs. These data indicate that FMD cycles have wide and differential effects on normal, cancer and cancer stem cells, allowing the rapid identification and targeting of starvation escape pathways and providing a method potentially applicable to many malignancies. **INSERT PAGE 2A**

### SUMMARY OF THE INVENTION

Note that the references to the methods of treatment by therapy in this description are to be interpreted as references to compounds, pharmaceutical compositions and medicaments of the present invention for use in those methods.

In the present invention, the authors have identified that the combination of a specific caloric intake regime with an optimized chemotherapeutic treatment is effective in the treatment of cancer, in particular in the treatment of triple negative breast cancer. Said specific caloric intake regime is based on a reduced daily caloric intake compared to a regular daily caloric intake, in particular it involves a specific daily caloric intake and a specific macronutrient intake as defined below.

The invention is based on the surprising finding that a reduced caloric intake or a fasting mimicking diet (FMD) enhances the therapeutic activity of anticancer agent in the treatment of cancer. Within the present invention it has been demonstrated that a fasting-mimicking diet (FMD) activates starvation escape pathways in triple negative breast cancer cells, which can be identified and targeted by drugs. In cancer stem cells, FMD cycles lower glucose-dependent WO 2015/134837 discloses a method for treating cancer comprising administering a reduced caloric diet to the subject with an anticancer drug and WO 2011/050302 discloses a method for treating cancer comprising at least one reduced caloric intake cycle and at least one anticancer agent. Publications De Groot et al (Nature communications, vol 11, no. 1, 1 December 2020, XP055835240) and Deligiorgi et al (International Journal of Molecular Sciences, vol 21, no. 23, 1 December 2020, p. 9175, XP055835415) describe FMD as an adjuvant to chemotherapy. Song Mengqiu et al. (Cancer research, vol. 79, no. 6, 15 Mrch 2019, p. 1019-1031, XP055835371) discloses the use of AKT inhibitors in the treatment of cancer. protein kinase A signalling and stemness markers to reduce cell number and increase mouse survival. Accordingly, metastatic triple negative breast cancer patients with lower glycemia survive longer than those with higher baseline glycemia. By contrast, in differentiated cancer cells, FMD cycles activate the PI3K-AKT, mTOR, and CDK4/6 pathways, targeted by drugs to promote tumour regression. FMD cycles also prevent the hyperglycemia and toxicity caused by these drugs. These data indicate that FMD cycles have wide and differential effects on normal, cancer and cancer stem cells, allowing the rapid identification and targeting of starvation escape pathways and providing a method potentially applicable to many malignancies.

It is therefore an object of the invention at least one reduced caloric intake cycle and at least one anticancer agent for use in the treatment of cancer wherein said at least one reduced caloric intake cycle provides at most 2100 Kcal per day and consists of a first and a second part, wherein the first part has a regular caloric intake reduced by 30% to 70% and the second part has a regular caloric intake reduced by 40 to 97%;
and wherein said at least one anticancer agent is
- the AKT inhibitor Ipatasertib

In a preferred embodiment the invention provides at least one reduced caloric intake cycle and at least one anticancer agent for use in the treatment of cancer wherein said at least one reduced caloric intake cycle provides at most 2100 Kcal per day and consists of a first and a second part, wherein the first part has a regular caloric intake reduced by 30% to 70% and the second part has a regular caloric intake reduced by 40 to 97%:
and wherein said at least one anticancer agent is
- the AKT inhibitor Ipatasertib

In a preferred embodiment, the invention provides the at least one reduced caloric intake cycle and at least one anticancer agent for use as defined above, wherein the at least one anticancer agent is
- the AKT inhibitor; and
further comprising at least one additional agent wherein said at least one additional agent is a PI3K inhibitor or a mTOR inhibitor or a CDK4/6 inhibitor.

Preferably the AKT inhibitor is Ipatasertib; the hexokinase inhibitor is 2-Deoxy-D-Glucose, tuvatexib, VDA-1275, 2-DG, KULA-19, lonidamine; the PI3K inhibitor is pictilisib, alpelisib, paxalisib, rigosertib, dactolisib, MEN-1611, pilaralisib, VT-30, ART-001, CHF-6523, CUDC-908, HEC-68498, SF-1126, WXFL-10030390, YZJ-0673, AL-58805, AUM-302; the mTOR inhibitor is Rapamycin, everolimus, temsirolimus, onatasertib, bimiralisib, dactolisib , monepantel, sapanisertib, vistusertib, CC-115, CERC-006, detorsertib, FP-208; the CDK4/6 inhibitor is Palbociclib, abemaciclib, ribociclib, Trilaciclib, alvocidib, ebvaciclib, lerociclib, milciclib, SHR-6390, AT-7519, AZD-4573, BEY-1107, BPI-1178, CT-7001, FCN-437c, FIT-039.

Preferably the AKT inhibitor is Ipatasertib, the hexokinase inhibitor is 2Deoxy-D-Glucose, the PI3K inhibitor is Pictilisib or Alpelisib, the mTOR inhibitor is Rapamycin, the CDK4/6 inhibitor is Palbociclib.

The invention further provides the least one reduced caloric intake and at least one anticancer agent for use according to anyone of previous claims, comprising administering a combination of:
- Ipatasertib and Rapamycin; or
- Ipatasertib and Pictilisib; or
- Ipatasertib and Alpelisib; or
- Ipatasertib and Rapamycin and Pictilisib; or
- Ipatasertib and Rapamycin and Alpelisib; or

Therefore, the invention provides at least one reduced caloric intake cycle and an anticancer agent for use in the treatment of cancer wherein said at least one reduced caloric intake cycle comprises a first part with a regular caloric intake reduced by 30% to 70% and a second part with a regular caloric intake reduced by 40 to 97%.

Preferably said first part and/or said second part lasts for a period of 24 to 190 hours, preferably said first part and/or or said second part lasts for a period of 24 to 120 hours, preferably said first part and/or or said second part lasts for approximately 120 hours.

Preferably the at least one reduced caloric intake cycle is repeated from 1 to 30 times after respective periods of from 5 to 60 days.

In other words, each cycle may be separated by 5 to 60 days.

In a further embodiment of the invention the at least one reduced caloric intake cycle and at least one anticancer agent is administered with a further therapeutic intervention.

Further therapeutic intervention is selected from the group consisting of: surgery, radiotherapy and at least one further therapeutic agent.

Preferably said further therapeutic agent is a further AKT inhibitor, hexokinase inhibitor, PI3K inhibitor, mTOR inhibitor or CDK4/6 inhibitor, an immune checkpoint inhibitor, an immune response stimulator, a targeted anticancer agent, a DNA Damage Response inhibitor and/or a chemotherapeutic agent.

Preferably said further immune checkpoint inhibitor is selected from the group consisting of: PD1 inhibitors, PDL1 inhibitors, CTLA-4 inhibitors, TIGIT inhibitors, ICOS inhibitors, TIM3 inhibitors, IDO1 inhibitors; said immune response stimulator is selected from the group consisting of: OX40 activators, GITR modulators, 4-1BB agonists; said targeted anticancer agent is selected from the group consisting of: PI3K inhibitors, HDAC inhibitors, EGFR inhibitors, BRAF inhibitors, MAPK inhibitors, CDK inhibitors, ER stress activators; said DNA Damage Response inhibitor is selected from the group consisting of: PARP inhibitors, CHK1 inhibitors, ATR inhibitors, Wee1 inhibitors; said chemotherapeutic agent is selected from the group consisting of: Alkylating agents, Antimetabolites, Anti-microtubule agents, Topoisomerase inhibitors, Cytotoxic antibiotics.

Preferably said cancer is a solid or hematopoietic cancer, preferably the cancer is selected from the group consisting of: breast cancer, triple negative breast cancer, melanoma, lymphoma, lung cancer, non-small cell lung cancer (NSCLC), head and neck cancer, gastroesophageal cancer, bladder cancer and urothelial cancer, hepatocellular carcinoma and renal cell carcinoma.

Preferably said cancer is triple negative breast cancer.

The cancer may be resistant to other agents such as commonly used chemotherapy.

Said cancer can be also characterized by resistance or partial response to the treatment with at least one immunotherapeutic agent.

In a non-claimed aspect, the present disclosure provides an in vitro method to identify starvation escape mechanisms in cancer cells, preferably in triple negative breast cancer cell (TNBC), comprising:
a. inoculating into a cancer cells thereby generating xenograft non-human animals, separating said xenograft non-human animals into two groups, a first group fed under ad libitum conditions (AL group) and a second group fed with fasting-mimicking diet (FMD group);
b. enzymatically digesting a sample of tumor mass from said AL group and FMD group;
c. sorting cancer cells of each digested sample for CD44CD24 human antibodies to separate, for each of the AL group and the FMD group, a first population of CD44^{high}CD24^{low} cells staminal and a second population of CD44^{high}CD24^{high} differentiated cells; and
d. identifying up and down regulated genes in said first and said second cell populations for each group by comparing: (a) gene expression of the CD44^{high}CD24^{low} cell staminal population of the digested sample from FMD group with the CD44^{high}CD24^{low} cell staminal population of the digested sample from AL group; and (b) gene expression of the CD44^{high}CD24^{high} differentiated cells of the digested sample from FMD group with the CD44^{high}CD24^{high} differentiated cells of the digested sample from AL group.

The present invention provides a method of treatment of cancer comprising:
- administering at least one reduced caloric intake cycle; and
- administering at least one anticancer agent as defined above
wherein said at least one reduced caloric intake cycle comprises a first part with a regular caloric intake reduced by 30% to 70% and a second part with a regular caloric intake reduced by 40 to 97% and wherein said at least one anticancer agent is the AKT inhibitor Ipatasertib.

Preferably said reduced caloric intake comprises a reduced protein intake and/or a reduced simple carbohydrate intake and/or an increased complex carbohydrate intake and/or an increased unsaturated fat intake.

Preferably said increased complex carbohydrate intake is approximately from 40% to 50% of total caloric intake, preferably said reduced protein intake is approximately from 9 to 11% of total caloric intake, preferably said increased complex carbohydrate intake is approximately from 43 to 47% of total caloric intake, preferably said increased unsaturated fat intake is approximately from 44 to 46% of total caloric intake.

In a preferred embodiment the reduced caloric intake is carried out by administering a specific regimen. Said regimen consists of a 4 days regimen. It provides approximately 1.100 kilocalories for a first day and less than 300 kilocalories per day for a second to fourth day of the diet (Table 1). It includes less than 30 grams of sugar on the first day; less than 5 grams of sugar on the second to fourth days; less than 30 grams of proteins on the first day; less than 5 grams of proteins on days the second to fourth days; less than 15 grams of saturated fats on the first day (Tables 2 and 3).

**Table 1. Exemplary of Fasting mimicking diet (FMD) developed to induce a fasting-like response while maximizing nourishment.**

| | **Day 1** | **Day 2** | **Day 3** | **Day 4** |
|---|---|---|---|---|
| **Total Calorie** | 1.112 | 240 | 238 | 153 |

**Table 2. The macronutrient content for each day of the 4 day FMD.**

| ***Average values.** | | |
|---|---|---|
| | **Day 1** | ***Day 2, 3, 4** |
| **Total Calorie** | 1.112 | ~ 210 |
| Fats | ~ 61 % | ~ 8% |
| Carbohydrates | ~ 31 % | ~ 80 % |
| *(of which sugars)* | (~ 9 %) | ~ 6% |
| Proteins | ~ 9% | ~ 5 % |

**Table 3. The micronutrient content for each day of the 4 day FMD regimen based on an average 180-200lbs person.**

| | **Unit** | **Day 1** | **Day 2,3,4*** |
|---|---|---|---|
| **Total Fats** | (g) | 75 | 2 |
| **% DV** | | 96 | 2,3 |
| **Proteins** | (g) | 25 | 2,6 |
| **% DV** | | 51 | 5 |
| **Total Carbohydrates** | (g) | 103 | 45,6 |
| **% DV** | | 37 | 13,3 |
| **Sugars** | (g) | 26,30 | 3,1 |
| **Dietary Fiber** | (g) | 32 | 5,7 |
| **% DV** | | 113 | 19,6 |
| **VIT A** | (IU) | 6.442 | 1.489 |
| **% DV** | | 716 | 157 |
| **VIT C** | (mg) | 9 | 15,6 |
| **% DV** | | 101 | 14.35 |
| **Calcium** | (mg) | 445 | 52,6 |
| **% DV** | | 34 | 5 |
| **Iron** | (mg) | 16 | 1,6 |
| **% DV** | | 91 | 8 |
| **Sodium** | (mg) | 1.970 | 1.148 |
| **% DV** | | 82 | 49,6 |
| **Potassium** | (mg) | 1.088 | 324,3 |
| **% DV** | | 23 | 6,6 |

| | | | |
|---|---|---|---|
| % DV indicates the percent of daily value based on a 2.000 calorie diet updated to 2020 regulations. *Average values | | | |

Embodiments and experiments illustrating the principles of the invention will be discussed with reference to the following figures.

### Brief description of the figures

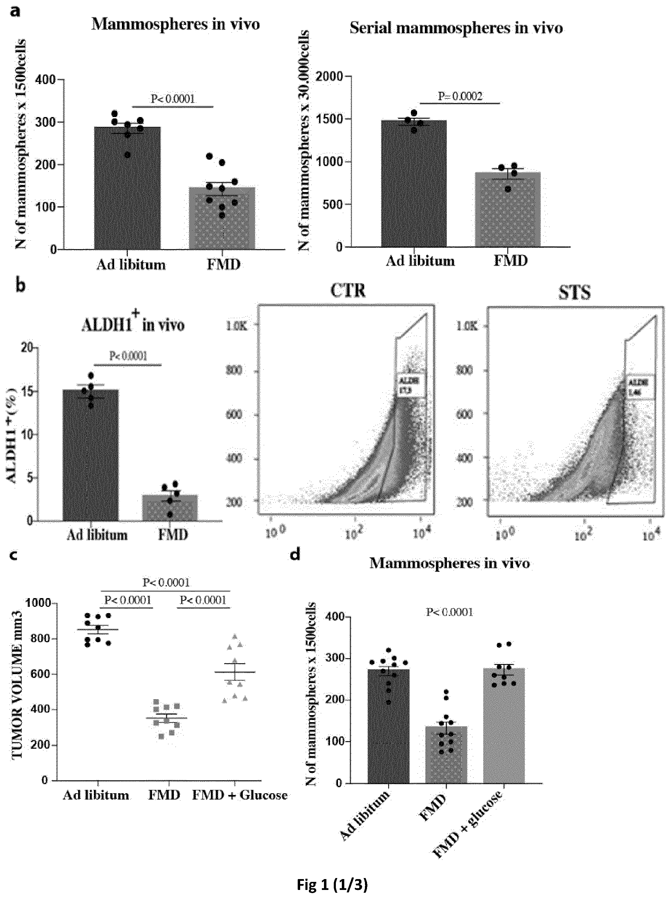

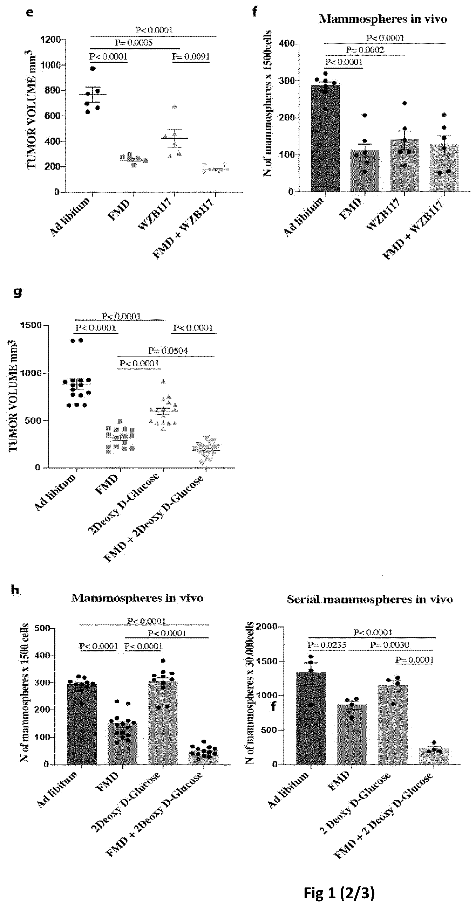

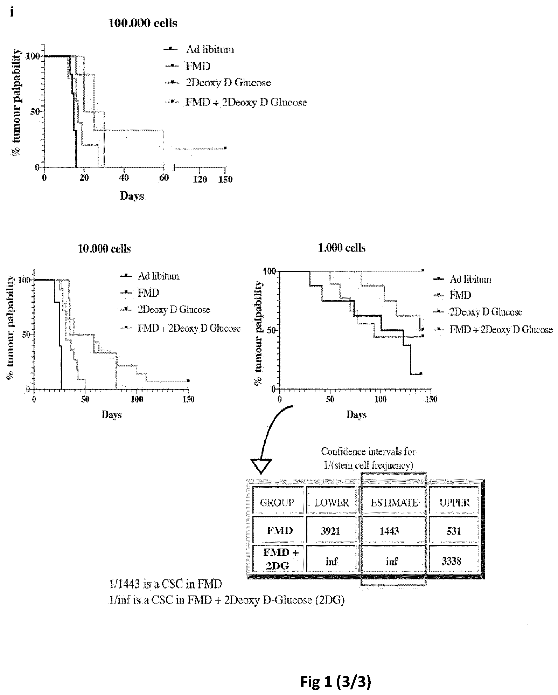

**Figure 1****. FMD reduces mammospheres growth, the expression of CSCs markers and stem cells frequency in SUM159 human triple negative breast cancer (TNBC), and its effect is potentiated by 2Deoxy-D-Glucose.** a) SUM159 tumour masses were processed for ex vivo primary mammosphere (n=7-9) and for ex vivo serial sphere forming assay (n=4). b) Aldefluor analysis were performed by flow cytometry using the ALDEFLUOR kit, to measure ALDH1 expression in SUM159 tumour masses (n=5). c) Growth of SUM159 xenografts in 8-weeks old female NOD scid (NSG) mice treated with AL diet or 5 cycles of FMD alone or plus 3%glucose supplementation in drinking water (n=9). d) Tumour masses were excised and processed for ex vivo primary mammospheres assay (n=9). e) Growth of SUM159 xenografts in 8-weeks old female NOD scid (NSG) mice treated with AL diet or 5 cycles of FMD alone or combined with WZB117 (10mg/kg) once a day, i.p (n=6). f) Tumour masses were excised and processed for ex vivo primary mammospheres assay (n=6). g) Growth of SUM159 xenografts in 8-weeks old female NOD scid (NSG) mice treated with AL diet or 5 cycles of FMD alone or combined with 2DG (200mg/kg) once a day, i.p. (n=15). h) Tumour masses were excised and processed for ex vivo primary mammospheres assay (n= 10-15) and serial mammospheres assay (n=5). i) SUM159 tumour cells derived from in vivo xenografts were injected in recipient mice at different dilution to perform the limiting dilution assay (n=6-14). P values were determined by Log-rank (Mantel-Cox) test (100.000cells: Ad libitum vs FMD, p= 0.0024; Ad libitum vs 2DG, p= 0.0660; Ad libitum vs FMD + 2DG, p= 0.0008; FMD vs 2DG, p= 0.1007; FMD vs FMD + 2DG, p= 0.1657; 2DG vs FMD + 2DG, p= 0.0177; 10.000 cells: Ad libitum vs FMD, p= 0.0011; Ad libitum vs 2DG, p= 0.0003; Ad libitum vs FMD + 2DG, p<0.0001; FMD vs 2DG, p= 0.0428; FMD vs FMD + 2DG, p= 0.3120; 2DG vs FMD + 2DG, p= 0.0192; 1.000 cells: Ad libitum vs FMD, p= 0.0891; Ad libitum vs 2DG, p= 0.3981; Ad libitum vs FMD + 2DG, p= 0.0001; FMD vs 2DG, p= 0.4714; FMD vs FMD + 2DG, p= 0.0123; 2DG vs FMD + 2DG, p= 0.0067). The stem cell frequency was calculated using ELDA software. In Fig. 1a-h, data are represented as mean ± SEM. P values were determined by two- tailed unpaired t test (Fig. 1a, b) and one-way Anova (Fig. 1c-h).

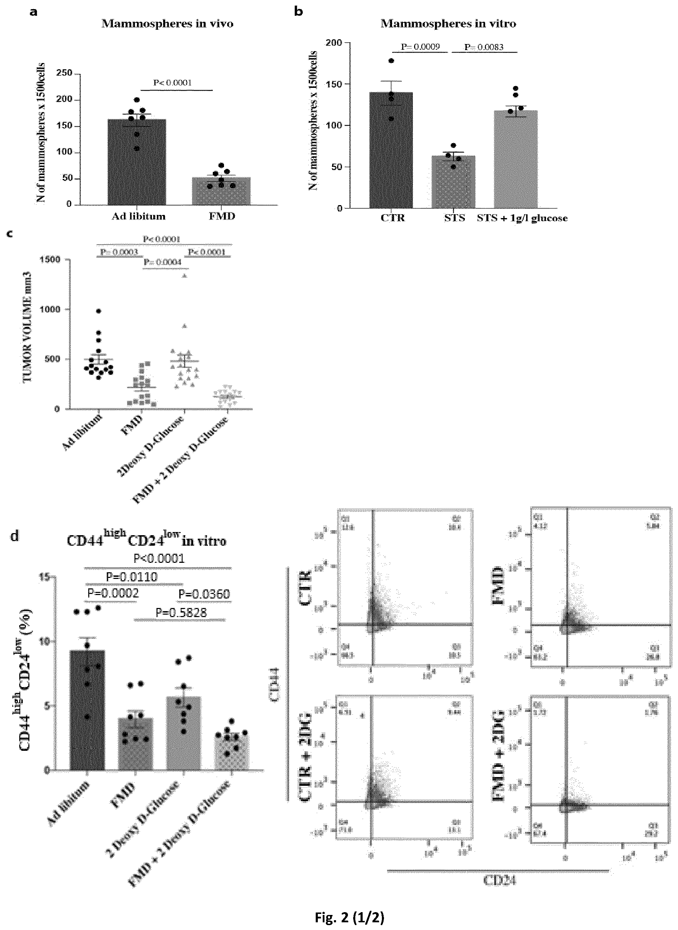

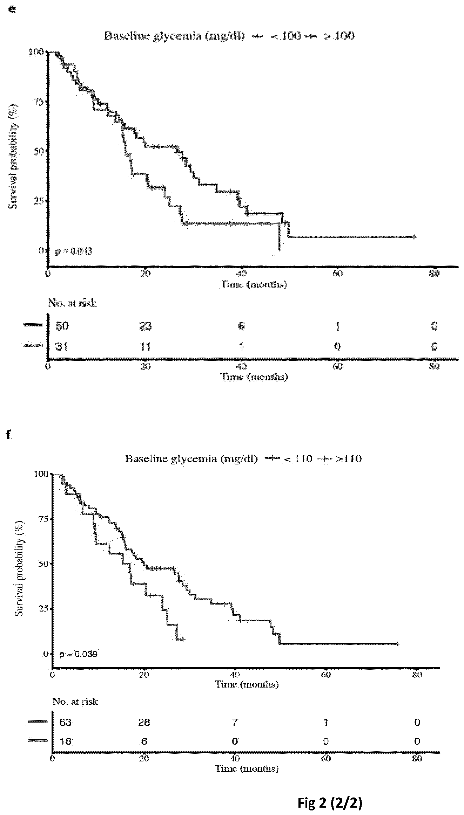

**Figure 2****. FMD acts independently of the immune system and low levels of baseline glycemia are important for the survival of patients with highly metastatic TNBC.** a) Growth of 4T1-luc cells in the mammary fat pad of 6-weeks old female Balb-c mice treated with AL diet or 4 cycles of FMD. Tumor masses were excised and processed for ex vivo primary mammosphere forming assay (n=7). b) 4T1 cells were grown under CTR (1g/l Glucose, 10%FBS), STS (0,5g/l, 1%FBS) and STS + 1g/l of glucose conditions for a total of 48h. Cells were then plated to perform the in vitro sphere forming assay (n=4 biological replicates). c) Growth of 4T1-luc cells in the mammary fat pad of 6-weeks old female Balb-c mice treated with AL diet or 4 cycles of FMD alone or combined with 2DG (200mg/kg) once a day, i.p. (n=15 per group). d) FACS analysis were performed to measure CD44 and CD24 expression in 4T1 TNBC in vivo (n=8). Data are represented as mean ± SEM. P values were determined by two- tailed unpaired t test (Fig. 2a) and one-way Anova (Fig. 2b-d). e-f) Kaplan-Meier curves for overall survival according to baseline blood glucose levels in advanced TNBC patients treated with first-line platinum-based doublet chemotherapy. Normoglycemia and hyperglycaemia were defined according to different thresholds: 100 mg/dl (e) and 110 mg/dl (f).

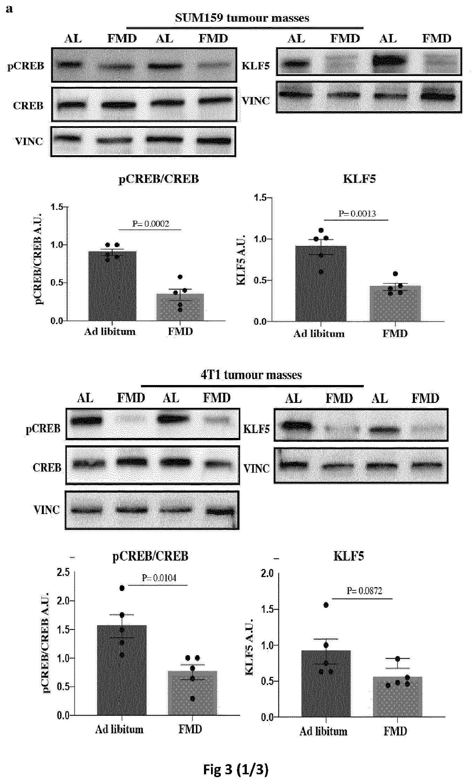

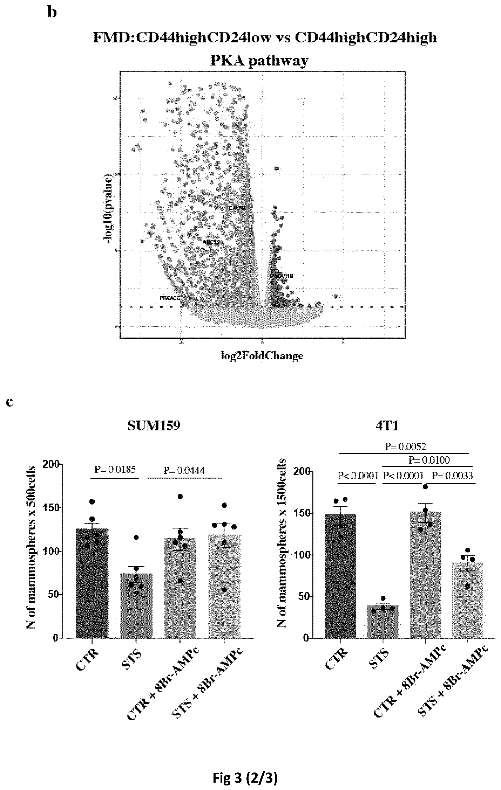

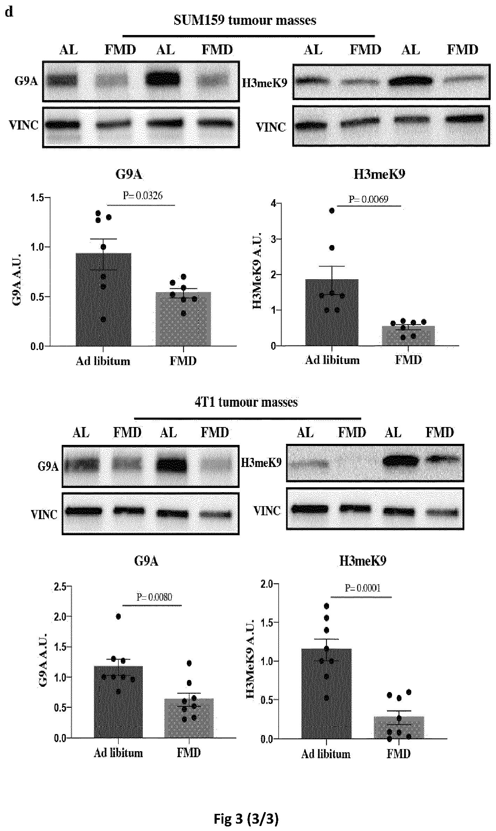

**Figure 3****. PKA is downregulated by FMD and its activation reverses STS dependent sphere reduction.** a) Detection of phosphorylated CREB levels, total CREB, KLF5 and VINCULIN, as loading control, in SUM159 and 4T1 tumor masses (n=5). b) Volcano plot showing the significance versus the log2 fold-change between CD44hiCD24lo and CD44loCD24hi. Up and downregulated genes (|log2FC| > 0.58 and adj. p value < 0.05) are displayed in red and green respectively. Deregulated genes involved in the PKA pathway are highlighted. c) SUM159 and 4T1 cells were grown under CTR (1g/l Glucose, 10%FBS) and STS (0,5g/l, 1%FBS) conditions for a total of 48h. At 24h cells were treated with 8-Br-cAMP. Cells were then plated to perform the in vitro spheres forming assay (n=4-6 biological replicates). d) Detection of G9A, H3meK9 levels and VINCULIN, as loading control, in SUM159 and 4T1 tumor (n=7-8). Data are represented as mean ± SEM. P values were determined by two- tailed unpaired t test (Fig. 3a, d) and one-way Anova (Fig. 3d).

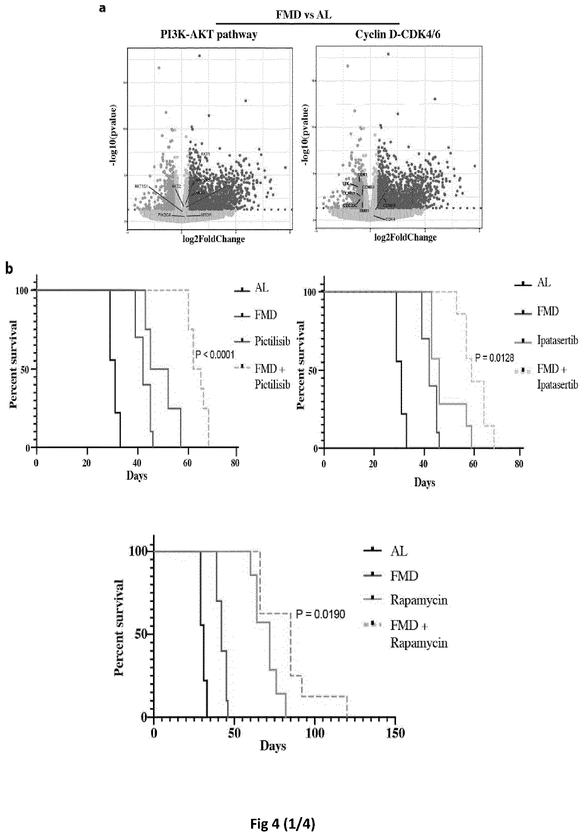

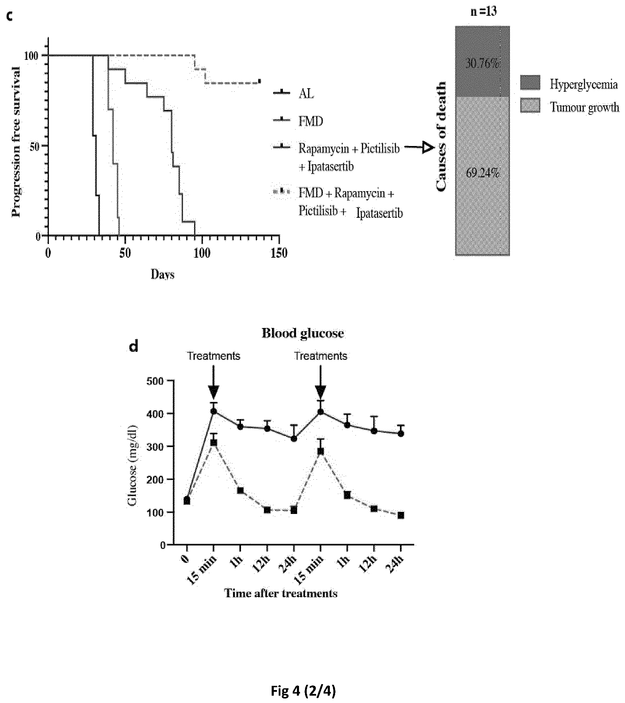

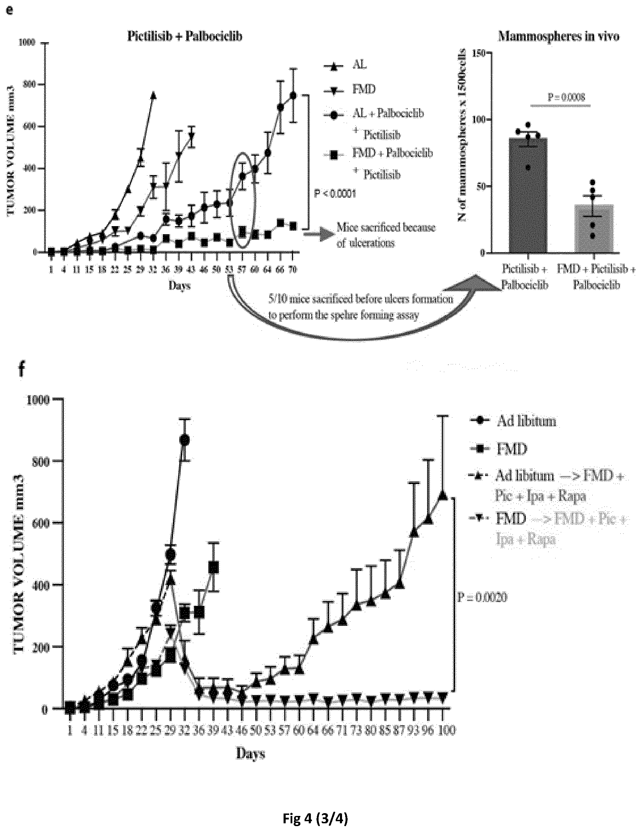

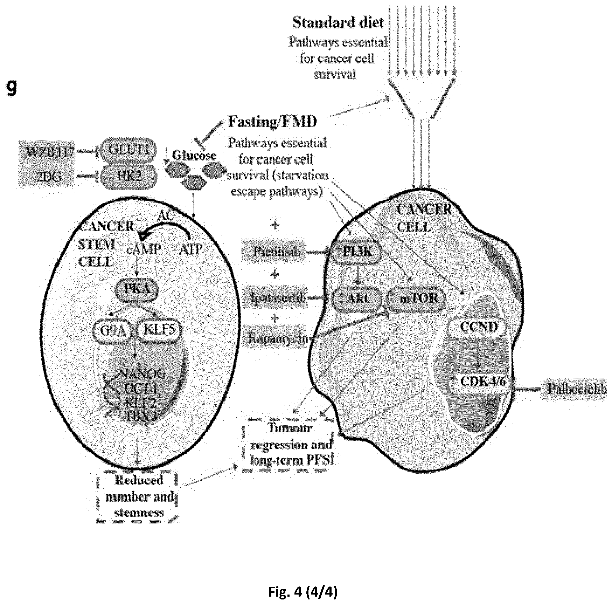

**Figure 4****. FMD reverts late stage-TNBC progression and protects from hyperglycemia side effects induced by PI3K pathway inhibitors.** a) Volcano plot showing the significance versus the log2 fold-change in SUM159 tumor masses, by comparing FMD versus AL. Up and downregulated genes (|log2FC| > 0.58 and adj. p value < 0.05) are displayed in red and green respectively. Deregulated genes involved in PI3K-AKT-mTOR pathway and CycD-CDK4/6 are highlighted. Significantly deregulated genes are reported in bold. b) Growth of SUM159 xenografts in 8-weeks old female NOD scid (NSG) treated with AL diet or FMD, alone or combined with pictilisib (100mg/kg, 5 consecutive days a week, by oral gavage), ipatasertib (75mg/kg, 5 consecutive days a week, by oral gavage) or rapamycin (2mg/kg, every other day, i.p.) (n=8-12). Survival curves are reported. c) Growth of SUM159 xenografts in 8-weeks old female NOD scid (NSG) mice treated with AL diet or FMD, alone or combined with pictilisib (100mg/kg, 5 consecutive days a week, by oral gavage), ipatasertib (75mg/kg, 5 consecutive days a week, by oral gavage) and rapamycin (2mg/kg, every other day, i.p.). n=10-13. Progression free survival was monitored over time and causes of death are reported. d) Blood glucose level was determined through Accu chek guide instrument. e) Growth of SUM159 xenografts in 8-weeks old female NOD scid (NSG) mice treated with Al diet or FMD, alone or combined with palbociclib (62,5 mg/kg, by oral gavage, every other day) and pictilisib (100 mg/kg, by oral gavage, 5 consecutive days a week) (n=10). Tumour masses were used to perform the *ex vivo* sphere forming assay (n=5). f) Growth of SUM159 xenografts in 8-weeks old female NOD scid (NSG) mice treated with AL diet or FMD. 35 days post cells injection mice started to be treated with pictilisib (100mg/kg, 5 consecutive days a week, by oral gavage), ipatasertib (75mg/kg, 5 consecutive days a week, by oral gavage) and rapamycin (2mg/kg, every other day, i.p.) plus FMD (n=15). g) Putative model for the effect of FMD on SUM159 TNBC cells and CSCs. P values were determined by Log-rank (Mantel-Cox) test (Fig. 4b, c) and two-tailed unpaired t test (Fig. 4e). Data are represented as mean ± SEM.

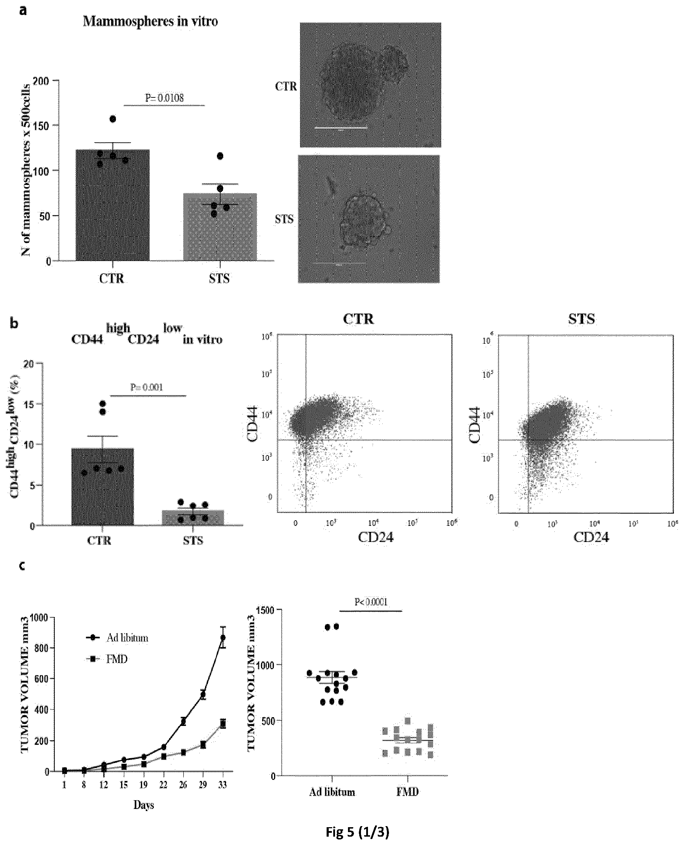

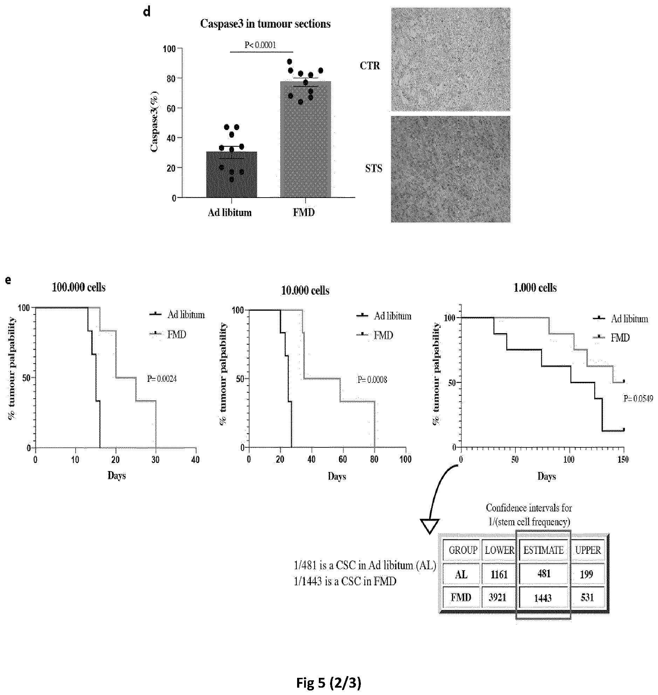

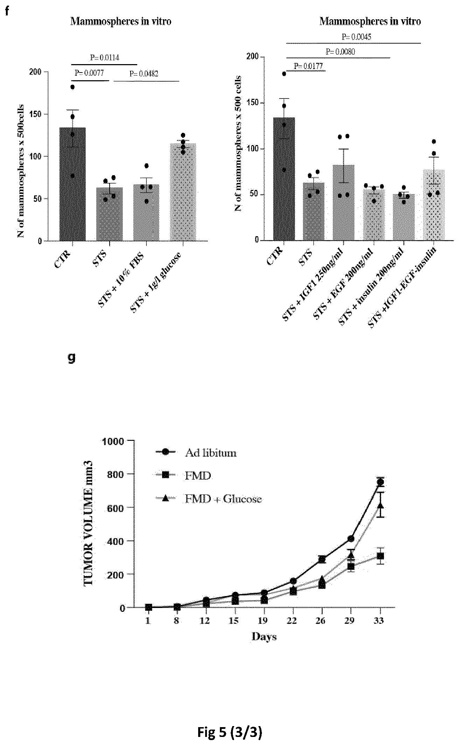

**Figure 5****.** a) SUM159 cells were grown under control (CTR: 1g/l Glucose, 10%FBS) and starved (STS: 0,5g/l, 1%FBS) conditions for a total of 48h. Cells were then plated to perform the sphere forming assay. The number and the morphology of representative SUM159 spheres, after 8 days of *in vitro* culture (n= 5 biological replicates) are shown. b) FACS analysis were performed to measure CD44 and CD24 expression in SUM159 breast cancer cell line in vitro (n= 6 biological replicates). c) Growth of SUM159 xenografts in 8-weeks old female NOD scid (NSG) treated with AL diet or 5 cycles of FMD (n=15). d) The expression of Cas-3 protein was examined by IHC staining in SUM159 tumour masses slides (n= 10, slides of different tumours, per group). Cas-3 positive cells were quantified with cell counter ImageJ plugin. e) SUM159 tumour cells derived from in vivo xenografts were injected in recipient mice at different dilution to perform the limiting dilution assay (n=6-8). The stem cell frequency was calculated using ELDA software. P values were determined by Log-rank (Mantel-Cox) test. f) SUM159 cells were grown under CTR (1g/l Glucose, 10%FBS), STS (0,5g/l, 1%FBS), STS + 1g/l glucose-1%FBS, STS + 10%FBS-0,5g/l glucose and STS + 0,5g/l glucose-1%FBS and single FBS components at CTR concentration level (IGF-1: 250ng/ml, EGF: 200ng/ml, Insulin: 200ng/ml) for a total of 48h. Cells were then plated to perform the in vitro sphere forming assay (n=4 biological replicates). g) Growth of SUM159 xenografts in 8-weeks old female NOD scid (NSG) mice treated with AL diet or 5 cycles of FMD alone or plus 3% glucose supplementation in drinking water (n=9). Data are represented as mean ± SEM. P values were determined by two-tailed unpaired t test (Fig. 5a-d) and one-way Anova (Fig. 5f).

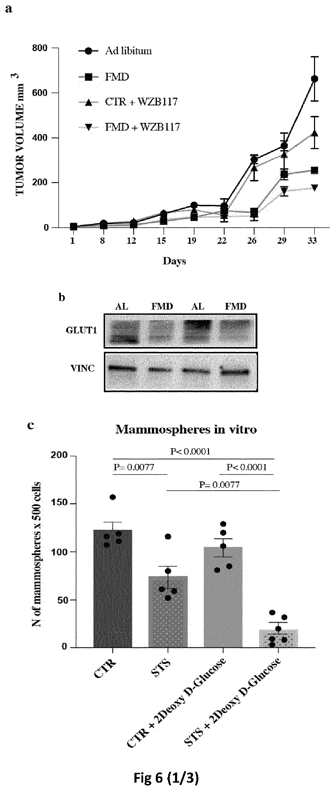

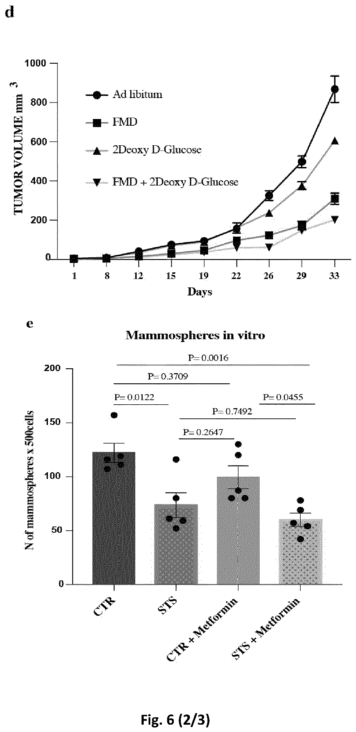

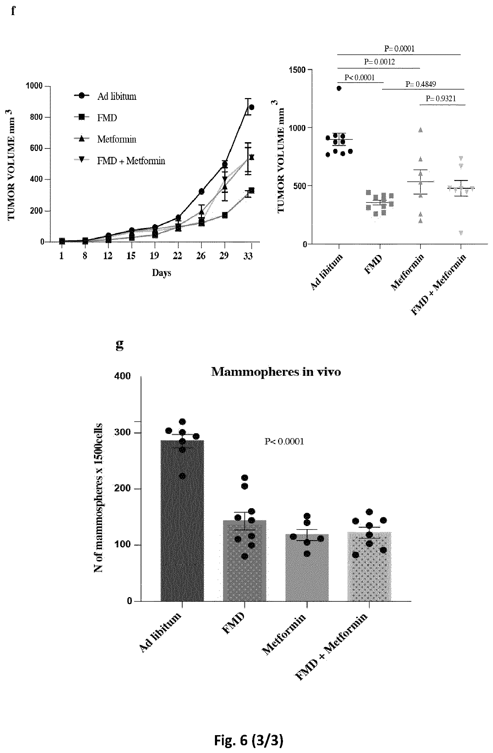

**Figure 6****.** a) Growth of SUM159 xenografts in 8-weeks old female NOD scid (NSG) mice treated with AL diet or 5 cycles of FMD alone or combined with WZB117 (10mg/kg) once a day, i.p. (n=6). b) Detection of GLUT1 levels and VINCULIN, as loading control, in SUM159 xenografts. c) SUM159 cells were grown under control (CTR: 1g/l Glucose, 10%FBS) and starved (STS: 0,5g/l, 1%FBS) conditions for 48h, and then treated with placebo or 2DG (4mM). Cells were then plated to perform the sphere forming assay (n=5 biological replicates). d) Growth of SUM159 xenografts in 8-weeks old female NOD scid (NSG) mice treated with AL diet or 5 cycles of FMD alone or combined with 2DG (500mg/kg) once a day, i.p (n=15-16). e) SUM159 cells were grown under control (CTR: 1g/l Glucose, 10%FBS) and starved (STS: 0,5g/l, 1%FBS) conditions for 48h, and then treated with placebo or metformin (5mM). Cells were then plated to perform the sphere forming assay (n=5 biological replicates). f) Growth of SUM159 xenografts in 8-weeks old female NOD scid (NSG) mice treated with AL diet or 5 cycles of FMD alone or combined with metformin (150mg/kg) once a day, i.p. (n=7-10). g) Tumour masses were excised and used to perform the *ex vivo* sphere forming assay (n=7-9). Data are represented as mean ± SEM. P values were determined by one-way Anova.

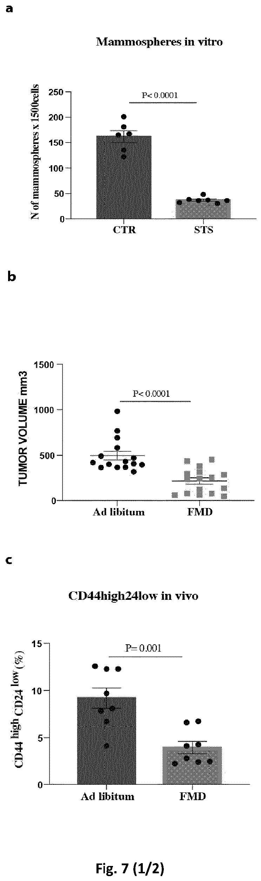

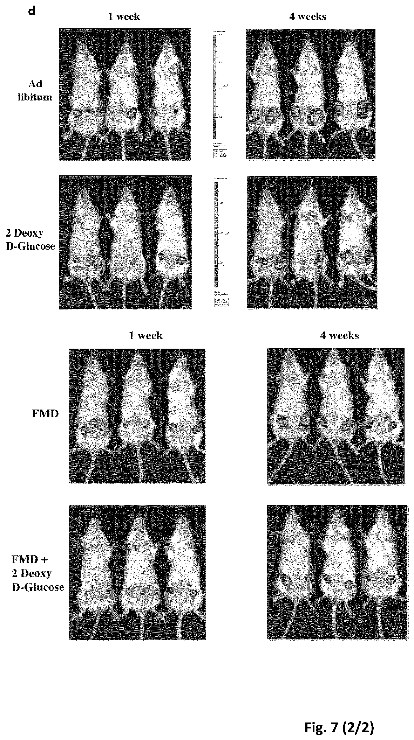

**Figure 7****.** a) 4T1 cells were grown under control (CTR: 1g/l Glucose, 10%FBS) and starved (STS: 0,5g/l, 1%FBS) conditions for a total of 48h. Cells were then plated to perform the sphere forming assay. b) Growth of 4T1 xenografts in 6-weeks old female Balb-c mice treated with AL diet or subjected to 4 cycles of FMD. (n=15-16 per group). c) FACS analysis were performed to measure CD44 and CD24 expression in 4T1 xenografts (n= 8). d) Growth of 4T1 xenografts in 6-weeks old female Balb-c mice treated with AL diet or subjected to 4 cycles of FMD, alone or combined with 2DG (500mg/kg) once a day, i.p. Tumour progression was monitored with bioluminescent imaging 1 week and 4 weeks after 4T1-luc cells injection in the mammary fat pad. Data are represented as mean ± SEM. P values were determined by two tailed unpaired t test.

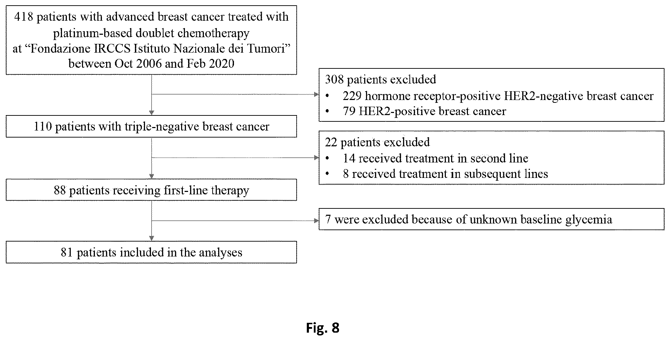

**Figure 8****.** CONSORT flow diagram showing the selection process leading to the identification of patients with advanced TNBC treated at Fondazione IRCCS Istituto Nazionale dei Tumori treated with first-line platinum-based chemotherapy.

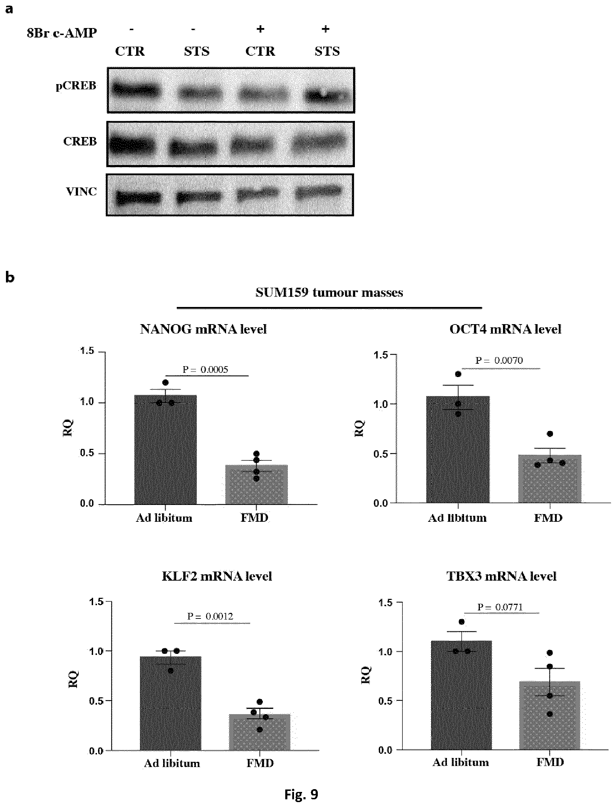

**Figure 9****.** a) Detection of phosphorylated CREB levels, total CREB and VINCULIN, as loading control, in SUM159 cells after 48h in STS medium (0,5 g/L glucose and 1% FBS), alone or in combination with 8-Br-cAMP. b) qPCR analysis was performed on SUM159 xenografts bared by mice fed with FMD or standard diet (CTR) (n=3-4). Data are represented as mean ± SEM. P values were determined by two tailed unpaired t test.

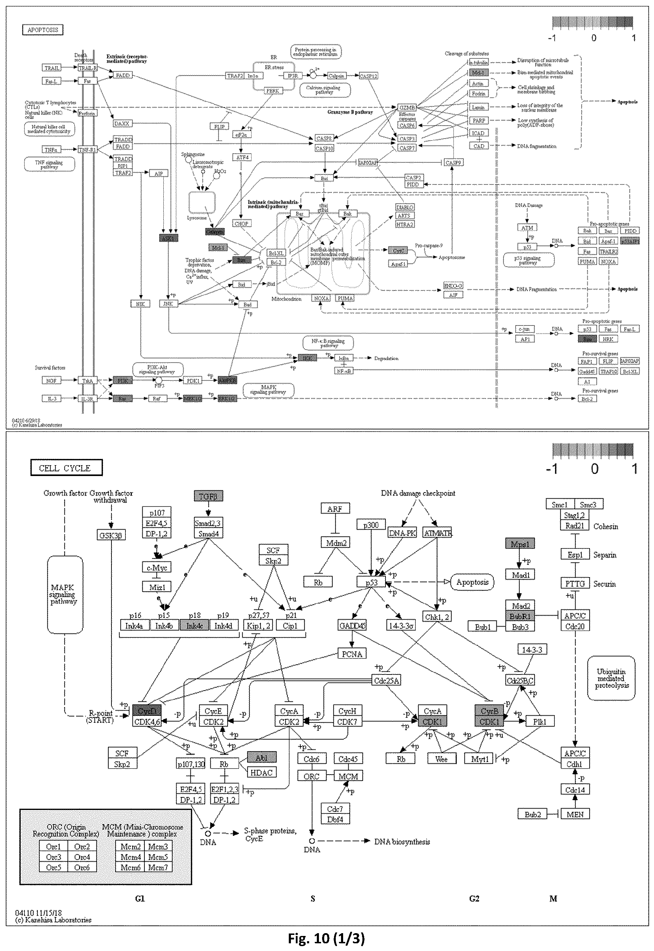

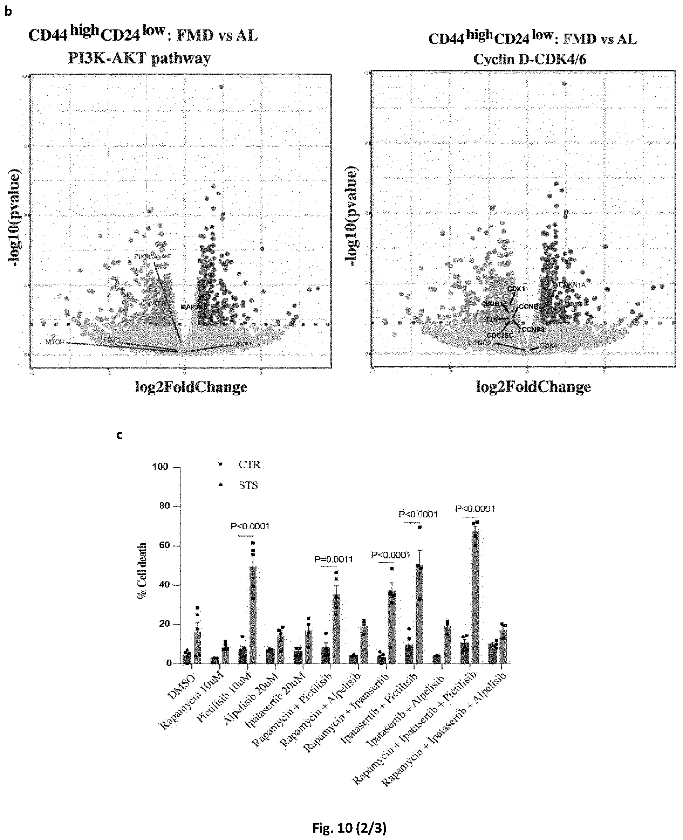

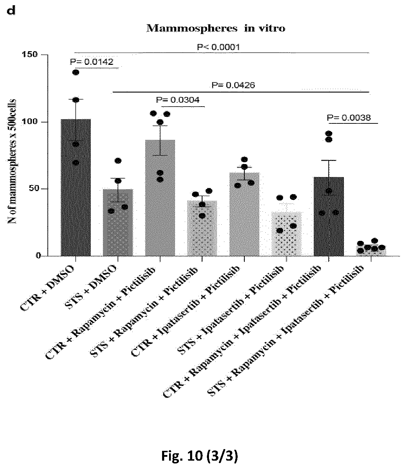

**Figure 10****.** a) Graph representation of KEGG apoptosis (hsa04210) and KEGG cell cycle (hsa04110). Significantly up and downregulated genes (by comparing FMD versus AL) are depicted in red and green respectively. b) Volcano plot showing the significance versus the log2 fold-change in SUM159 tumor masses, in CD44^{high}CD24^{low} population, by comparing FMD versus AL. Up and downregulated genes (|log2FC| > 0.58 and adj. p value < 0.05) are displayed in red and green respectively. Deregulated genes involved in PI3K-AKT-mTOR pathway and CycD-CDK4/6 are highlighted. Significantly deregulated genes are reported in bold. c) SUM159 cells were grown under CTR (1g/l Glucose, 10%FBS) and STS (0,5g/l, 1%FBS) conditions for a total of 48 hours. At 24h cells were treated with rapamycin (10µM), pictilisib (10µM), alpelisib (20µM) and ipatasertib (20µM), for 24hours, as single, double or triple treatments. Viability was assessed with erythrosine stain (n= 4-5 biological replicates), or d) cells were plated to perform the sphere forming assay (n=4-6 biological replicates). Data are represented as mean ± SEM. P values were determined by one-way Anova.

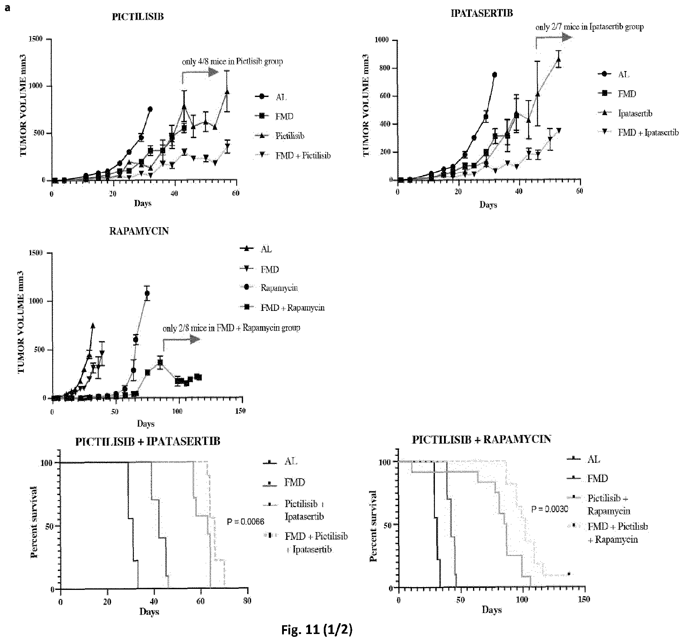

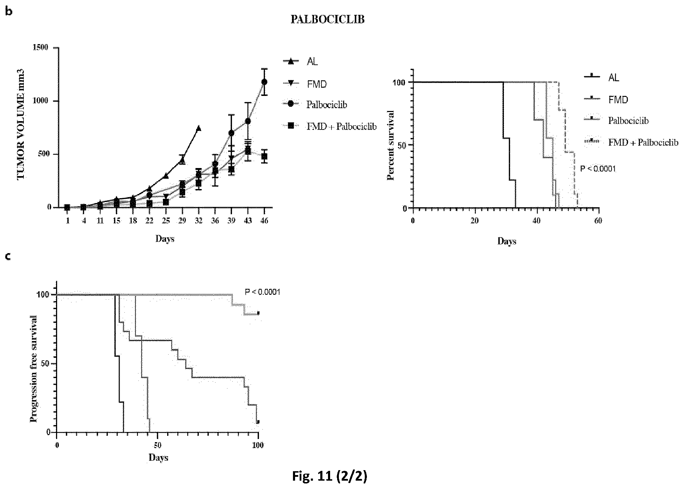

**Figure 11****.** a) Growth of SUM159 xenografts in 8-weeks old female NOD scid (NSG) mice treated with AL diet FMD, alone or combined with pictilisib (100mg/kg, 5 consecutive days a week, by oral gavage), ipatasertib (75mg/kg, 5 consecutive days a week, by oral gavage) or rapamycin (2mg/kg, every other day, i.p.) (n=8-12). Survival curves for pictilisib + ipatasertib and pictilisib + rapamycin are reported. b) Growth of SUM159 xenografts in 8-weeks old female NOD scid (NSG) mice treated with AL diet or FMD, alone or combined with palbociclib (62,5 mg/kg) every other day, by oral gavage. Survival curve is reported. c) Progression free survival related to experiment in Fig. 4f was monitored over time. P values were determined by Log-rank (Mantel-Cox) test.

### DETAILED DESCRIPTION OF THE INVENTION

The present invention provides a reduced caloric intake and at least one anticancer agent for the treatment of cancer. The invention provides at least one reduced caloric intake cycle and at least one anticancer agent for use in the treatment of cancer wherein said at least one reduced caloric intake cycle provides at most 2100 Kcal per day and consists of a first and a second part, wherein the first part has a regular caloric intake reduced by 30% to 70% and the second part has a regular caloric intake reduced by 40 to 97%;
and wherein said at least one anticancer agent is the AKT inhibitor Ipatasertib.

The reduction is compared to a regular caloric intake per day. Regular caloric intake per day is between 1200 Kcal and 3000 Kcal. Preferably regular caloric intake per day (the range is based on age, sex and physical activity) is:
Age 4-8 years: 1200-2000 Kcal Age 9-13 years: 1800-2600 Kcal Age 19-30 years: 1800-3000
Kcal Age 31-50 years: 1800-2600 Kcal
+51 years: 1600-2600 Kcal.

In an embodiment, the reduced caloric intake for use according to the present invention lasts for a period of 24 to 190 hours, preferably said reduced caloric intake lasts for a period of 24 to 120 hours, preferably said reduced caloric intake lasts for approximately 120 hours.

In a preferred embodiment the period of reduced caloric intake is of 48 to 168 hours, preferably 120 hours.

Preferably the reduced caloric intake starts at least 24 hours before the anticancer agent is administered. Preferably the reduced caloric intake starts at least 48 hours before the anticancer agent is administered. Preferably the reduced caloric intake lasts at least 24 hours after the anticancer agent is administered, preferably it lasts at least 48, 72, 96, 120 hours after the anticancer agent is administered.

Preferably the reduced caloric intake is started one day before the anticancer agent is administered and continues for the following 2-4 days after anticancer agent administration (i.e. while the anticancer agent is most active). Preferably the reduced caloric intake consists of 4 days of low-calorie intake (50% of regular calorie intake on day 1, and 10% on days 2-4).

In the present invention, preferably, the reduced caloric intake is obtained by fasting or by means of dietetic food with reduced caloric and/or protein content but containing all necessary micronutrients to prevent malnutrition.

In the present invention, preferably, the reduced caloric intake is obtained by fasting mimicking diet (FMD). The term "fasting mimicking diet" (FMD) means a diet that mimics the effects of fasting typically by providing a subject with at most 50% of his normal caloric intake but with some nutritional component, so that fasting is mimicked while a subject is not completely starved. Examples of useful fasting mimicking and enhancing in the context of the present invention are set forth in U.S. Pat. Appl. Nos. 14/273946 filed May 9, 2014; 14/497752 filed September 26, 2014; 12/910508 filed October 22, 2010; 13/982307 filed February 8, 2012; 14/060494 filed October 22, 2013; 14/178953 filed February 12, 2014; 14/320996 filed July 1, 2014; 14/671622 filed March 27, 2015. Additional examples of FMD diets are found in U.S. Pat. Appl. No. 15/148,251 and WIPO Pub. No. WO 2011/050302 and WIPO Pub. No. WO 2011/050302.

In the present invention the reduced caloric intake period is repeated one or more times after respective periods of 5-60 days, during which said mammal is given the agent while being subjected to a diet involving a regular caloric intake.

Preferably, the reduced caloric intake period is repeated from 1 to 3 times after respective period of 5-60 days.

In a preferred embodiment, the reduced caloric intake and at least one anticancer agent for use according to the invention are combined with a further therapeutic intervention selected from the group consisting of: surgery, radiotherapy and at least one further therapeutic agent.

Table 4 shows a list of drug classes that can be used in combination with the reduced caloric intake and the at least one anticancer agent for use according to the invention.

**Table 4. Drug classes that can be used in combination with the reduced caloric intake and the at least one anticancer agent.**

| **Drug Class** | **Drugs** | **Examples** |
|---|---|---|
| Immune checkpoint inhibitors | PD1 inhibitors | anti-PD1 monoclonal antibodies |
| | PDL1 inhibitors | anti-PDL1 monoclonal antibodies |
| | CTLA-4 inhibitors | anti-CTLA4 monoclonal antibodies |
| | TIGIT inhibitors | anti-TIGIT monoclonal antibodies |
| | ICOS inhibitors | anti-ICOS monoclonal antibodies |
| | TIM3 inhibitors | anti-TIM3 monoclonal antibodies |
| | IDO1 inhibitors | Epacadostat monoclonal antibodies |
| Immune response stimulators | OX40 activators | anti-OX40 monoclonal antibodies |
| | GITR modulators | anti-GITR monoclonal antibodies |
| | 4-1BB agonists | anti-4-1BB monoclonal antibodies |
| Targeted anticancer agent | PI3K inhibitors | Buparlisib |
| | HDAC inhibitors | Entinostat |
| | EGFR inhibitors | Cetuximab |
| | BRAF inhibitors | Vemurafenib |
| | MAPK inhibitors | Dabrafenib |
| | CDK inhibitors | Palbociclib |
| | ER stress activators | Honokiol |
| DNA Damage Response Inhibitors | PARP inhibitors | Olaparib |
| | CHK1 inhibitors | LY2603618 |
| | ATR inhibitors | AZD6738 |
| | Wee1 inhibitors | AZD1775 |
| Chemotherapeutic Drugs | Alkylating agents | Cyclophosphamide |
| | Antimetabolites | 5-fluorouracil |
| | Anti-microtubule agents | Paclitaxel |
| | Topoisomerase inhibitors | Camptothecin |
| | Cytotoxic antibiotics | Bleomycin |

In a preferred embodiment, the reduced caloric intake and at least anticancer agent for use according to the invention are combined with a further therapeutic agent is a further immune checkpoint inhibitor, an immune response stimulator, a targeted anticancer agent, a DNA Damage Response inhibitor and/or a chemotherapeutic agent.

In a preferred embodiment, the reduced caloric intake and at least one anticancer agent for use according to the invention are combined a further immune checkpoint inhibitor selected from the group consisting of: PD1 inhibitors, PDL1 inhibitors, CTLA-4 inhibitors, TIGIT inhibitors, ICOS inhibitors, TIM3 inhibitors, IDO1 inhibitors, and/or an immune response stimulator selected from the group consisting of: OX40 activators, GITR modulators, 4-1BB agonists, and/or a targeted anticancer agent selected from the group consisting of: PI3K inhibitors, HDAC inhibitors, EGFR inhibitors, BRAF inhibitors, MAPK inhibitors, CDK inhibitors, ER stress activators, and/or a DNA Damage Response inhibitor selected from the group consisting of: PARP inhibitors, CHK1 inhibitors, ATR inhibitors, Wee1 inhibitors, and/or a chemotherapeutic agent selected from the group consisting of: Alkylating agents, Antimetabolites, Anti-microtubule agents, Topoisomerase inhibitors, Cytotoxic antibiotics. Non limitative examples of immune checkpoint inhibitors include: nivolumab, pembrolizumab, cemiplimab, camrelizumab, sintilimab, toripalimab, tislelizumab, atezolizumab, durvalumab, avelumab, APL-502, bintrafusp alfa, ipilimumab, tremelimumab, zalifrelimab, AK-104, BMS-986207, tiragolumab, AB-154, AMG-570, ELN-21, ELN-12, darvadstrocel, indoximod, epacadostat.

Examples of immune response stimulators include: BMS-986178, GSK-3174998, INCAGN-1949, KHK-4083, ABBV-368, ATOR-1015, Utomilumab.

Non limitative examples of targeted anticancer agents include: alpelisib, copanlisib, rigosertib, dactolisib, duvelisib, entinostat, romidepsin, sodium phenylbutyrate, belinostat, panobinostat, osimertinib, cetuximab, panitumumab, erlotinib, vemurafenib, sorafenib, regorafenib, dabrafenib, miltefosine, brimapitide, acumapimod, palbociclib, abemaciclib, ribociclib, alvocidib, honokiol.

Examples of DNA Damage Response inhibitors include: olaparib, rucaparib camsylate, talazoparib, niraparib, LY2603618, AZD7762, SRA-737, CBP-501, ESP-01, prexasertib, AZD6738, berzosertib, M-4344, BAY-1895344, AZD1775, adavosertib, ZNC-3. Non limitative examples of chemotherapeutic agents include: Cisplatin, Carboplatin, Dicycloplatin, Oxaliplatin, Picoplatin, Satraplatin, Cyclophosphamide, Chlormethine, Uramustine, Melphalan, Chlorambucil, Ifosfamide, Bendamustine, Carmustine, Lomustine, Streptozocin, Busulfan, Procarbazine, Altretamine, Dacarbazine, Temozolomide, Mitozolomide.

Preferably, the reduced caloric intake and the at least one anticancer agent according to the present invention can be used in the treatment of a cancer characterized by resistance or partial response to the treatment with at least one therapeutic agent.

Preferably, the reduced caloric intake and the at least one anticancer agent according to the present invention can be used in the treatment of a cancer characterized by a reduction in tumour mass or volume comprised between 0% and 90% after a treatment with at least one therapeutic agent.

Preferably, the reduced caloric intake and the at least one anticancer agent according to the present invention can be used in the treatment of a cancer characterized by a reduction in tumour mass or volume comprised between 0% and 90% after a treatment with at least one therapeutic agent selected from the group consisting of: an AKT inhibitor, an hexokinase inhibitor, a combination of a PI3K inhibitor and a CDK4/6 inhibitor, or a combination of a mTOR inhibitor and a PI3K inhibitor.

AKT inhibitors are compounds inhibiting the AKT serine/threonine kinase, also known as protein kinase B (PKB), is an oncogenic protein that regulates cell survival, proliferation, growth, apoptosis, and glycogen metabolism. Direct synthetic AKT inhibitors are MK-2206, AZD5363, GSK690693, GDC-0068, GSK2141795, GSK2110183, AT7867, CCT128930, BAY1125976, perifosine, AKT inhibitor VIII, and AKT1 and AKT-IN-1 and natural inhibitors, [6]-shogaol, herbacetin, and oridonin. Ipatasertib (GDC-0068) is an ATP-competitive pan-AKT inhibitor exerting antiproliferative and antisurvival effects against several cancer cell lines by inhibiting the PI3-K/ AKT pathway.

Hexokinase inhibitors target the first step of glucose metabolism catalyzed by hexokinase (HK), which phosphorylates glucose to form glucose-6-phosphate (G-6-P), which serves as a substrate for ATP production and metabolite biosynthesis. 2-DG inhibits glycolysis due to formation and intracellular accumulation of 2-deoxy-d-glucose-6-phosphate (2-DG6P), inhibiting the function of hexokinase and glucose-6-phosphate isomerase, and inducing cell death. Other inhibitors include 3-bromopyruvic acid, lonidamine, KHK-IN-2, D-Mannoheptulose and others.

A PI3K inhibitor is a class of medical drug that functions by inhibiting one or more of the phosphoinositide 3-kinase enzymes, which are part of the PI3K/AKT/mTOR pathway, an important signalling pathway for many cellular functions such as growth control, metabolism and translation initiation. Within this pathway there are many components, inhibition of which may result in tumor suppression. Small molecule inhibitors of PI3K include PI3K/mTOR inhibitors, pan-PI3K inhibitors, and isoform-selective PI3K inhibitors. PI3K inhibitors include: Pictilisib, Idelasib, Copanlisib, Duvelisib, Alpelisib, Umbralisib.

CDK4/6 inhibitors are compounds targeting the cyclin-dependent kinase 4 and 6 (CDK4/6). Known CDK 4/6 inhibitors include palbociclib, ribociclib, and abemaciclib.

mTOR inhibitors are a class of drugs that inhibit the mechanistic target of rapamycin (mTOR), which is a serine/threonine-specific protein kinase that belongs to the family of phosphatidylinositol-3 kinase (PI3K) related kinases (PIKKs). mTOR regulates cellular metabolism, growth, and proliferation by forming and signaling through two protein complexes, mTORC1 and mTORC2. The most established mTOR inhibitors are so-called rapalogs (rapamycin and its analogs), which have shown tumor responses in clinical trials against various tumor types.

Preferably, the reduced caloric intake and the at least one anticancer agent according to the present invention can be used in the treatment of a cancer selected from: breast cancer, melanoma, lymphoma, lung cancer, non-small cell lung cancer (NSCLC), head and neck cancer, gastroesophageal cancer, bladder cancer and urothelial cancer, hepatocellular carcinoma and renal cell carcinoma. Most preferably , the reduced caloric intake and the at least one anticancer agent according to the present invention can be used in the treatment of breast cancer, preferably triple negative breast cancer In a non-claimed aspect, the present disclosure provides an in vitro method of treating a cancer cell with at least one agent as defined in above, comprising:
- cultivating the cancer cell in a medium with reduced serum or glucose concentration; and
- treating the cancer cell with the anticancer agent.
wherein the serum concentration in the medium is less than 10% and the glucose concentration in less than 1 g/1, preferably the serum concentration is less than 5% , still preferably the serum concentration is 1% or less than 1% . Preferably the glucose concentration is less than 0.8 g/liter, preferably less than 0.6 g/liter, still preferably 0.5 g/liter, preferably less than 0.5 g/liter. Preferably the serum concentration in the medium is reduced by 10-90% or the glucose concentration in the medium is reduced by 20-90%, the reduction is in respect of normal or control concentrations (i.e. 10% of serum and 1 g/liter of glucose).

The present invention also provides a method of treatment of cancer comprising:
- administering a reduced caloric intake; and
- administering at least one anticancer agent.
wherein cancer is characterized by resistance or partial response to the treatment with at least one anticancer agent and wherein the at least one anticancer agent is the AKT inhibitor Ipatasertib.

Wherein the reduced caloric intake lasts for a period of 24 hours to 190 hours and wherein said reduced caloric intake is a daily caloric intake reduced by 10 to 100%.

**In** the present invention a preferred reduced caloric intake is as follows:
Day 1: 54% caloric intake, about 1,090 kcal (10% protein, 56% fat, 34% carbohydrate)
Days 2-7: 20-34% caloric intake, about 426-725 kcal (5.3-9% protein, 26-44% fat, 27.6-47% carbohydrate).

The at least one anticancer agent for use according to the present invention can be incorporated into pharmaceutical compositions. Such compositions typically include the at least anticancer agent and at least one pharmaceutically acceptable carrier. As used herein the wording "pharmaceutically acceptable carrier" includes saline, solvents, dispersion media, coatings, antibacterial and antifungal agents, isotonic and absorption delaying agents, and the like, compatible with pharmaceutical administration. Supplementary active compounds can also be incorporated into the compositions.

A pharmaceutical composition is formulated to be compatible with its intended route of administration. Examples of routes of administration include parenteral, e.g., intravenous, intradermal, subcutaneous, oral, inhalation, transdermal (topical), transmucosal, and rectal administration.

In certain preferred embodiments, the pharmaceutical composition comprising at least one anticancer agent for use according to the present invention, used for parenteral, intradermal, or subcutaneous application, may further comprise one or more pharmaceutically acceptable carriers, exemplified by, but not limited to, lipid particles, lipid vesicles, liposomes, niosomes, sphingosomes, polymeric nanocarriers, nanoparticles, microparticles, nanocapsules, and nanospheres.

The pharmaceutical compositions of the present invention are preferably in the form of a single unit dosage form that contains an amount of the therapeutic agent that is effective to treat and/or prevent a cancer of the type described herein and at least one pharmaceutically acceptable excipient.

Suitable pharmaceutically acceptable excipients are those commonly known to the person skilled in the art for the preparation of compositions for parenteral, intradermal, subcutaneous, oral, transdermal, topical, transmucosal, and rectal administration.

By way of example, said acceptable carriers can consists of binders, diluents, lubricants, glidants, disintegrants, solubilizing (wetting) agents, stabilizers, colorants, anti-caking agents, emulsifiers, thickeners and gelling agents, coating agents, humectants, sequestrants, and sweeteners.

The amount of the at least one anticancer agent in the pharmaceutical composition of the present invention will depend upon the nature and severity of the condition being treated, and on the nature of prior treatments which the patient has undergone. For a human patient, the attending physician will decide the dose of compound of the present invention with which to treat each individual patient. Initially, the attending physician can administer low doses and observe the patient's response. Larger doses may be administered until the optimal therapeutic effect is obtained for the patient, and at that point the dosage is not increased further. The duration of therapy using the pharmaceutical composition of the present invention will vary, depending on the severity of the disease being treated and the condition and potential idiosyncratic response of each individual patient.

In an embodiment, the invention provides a reduced caloric intake and at least one anticancer agent according to claim 1 for use in the treatment of cancer, wherein the cancer is characterized by resistance on partial response to other therapeutics. In addition the reduced caloric intake and the at least one anticancer agent according to the present invention are useful to prevent the hyperglycemia and toxicity caused by the anticancer agent.

### Definitions

According to the present invention, preventing a disease refers to inhibiting completely, or in part, the development or progression of a disease, for example in a person who is known to have a predisposition to a disease. An example of a person with a known predisposition is someone with a history of cancer in the family, or who has been exposed to factors that predispose the subject to the development of a tumour.

Treating a disease refers to a therapeutic intervention that inhibits, or suppressed the growth of a tumour, eliminates a tumour, ameliorates at least one sign or symptom of a disease or pathological condition, or interferes with a pathophysiological process, after the disease or pathological condition has begun to develop.

Therapeutically effective dose is used in the context of the present invention to characterize an amount of the drug, which leads to complete or partial remission of the neoplastic disease. For instance, any statistically significant reduction in the mass or volume of the tumour or in the number of cancer cells indicates therapeutic efficacy in the context of the present invention.

Pharmaceutically acceptable is used in the context of the present invention to refer to those compounds, materials, compositions, and/or dosage forms that are, within the scope of sound medical judgment, suitable for use in contact with the tissues of human beings and animals without excessive toxicity, irritation, allergic response, and/or other problem or complication, commensurate with a reasonable benefit/risk ratio.

Resistance is used in the context of the present invention to refer to no variation in tumour mass or volume after a treatment with at least one therapeutic.

Partial response is used in the context of the present invention to refer to a decrease in the size of a tumour, or in the extent of cancer in the body, in response to treatment with at least one therapeutic agent. Preferably, partial response is used in the context of the present invention to refer to a reduction in tumour mass or volume comprised between 0% and 90% after a treatment with at least one therapeutic agent. Partial response can also be called partial remission.

Immunotherapy is used in the context of the present invention to refer to the prevention or treatment of disease with agents (i.e. immunotherapeutic agents) that stimulate the immune response.

According to the invention, a anticancer agent is a drug or a combination of drugs to be used in cancer chemotherapy. In modern oncology the chemotherapeutics are chemotherapy regimens combining several chemotherapy drugs in combination chemotherapy. A fundamental philosophy of medical oncology, including combination chemotherapy, is that different drugs work through different mechanisms, and that the results of using multiple drugs will be synergistic to some extent. Because they have different dose-limiting adverse effects, they can be given together at full doses in chemotherapy regimens.

According to the present invention, simultaneous administration may, e.g., take place in the form of one fixed combination with two or more active ingredients, or by simultaneously administering two or more active ingredients that are formulated independently. Sequential use (administration) preferably means administration of one (or more) components of a combination at one time point, other components at a different time point, that is, in a chronically staggered manner, preferably such that the combination shows more efficiency than the single compounds administered independently (especially showing synergism). Separate use (administration) preferably means administration of the components of the combination independently of each other at different time points.

Also combinations of two or more of sequential, separate and simultaneous administration are possible, preferably such that the combination component-drugs show a joint therapeutic effect that exceeds the effect found when the combination component-drugs are used independently at time intervals so large that no mutual effect on their therapeutic efficiency can be found, a synergistic effect being especially preferred.

### MATERIALS AND METHODS

### Cell lines and culture conditions

The human triple negative breast cancer (TNBC) cell line SUM159 was purchased from Asterand (Asterand, Detroit, MI); the murine TNBC cell line 4T1 (ATCC^{®} CRL-2539^{™}) was purchased from ATCC. SUM159 cells were cultured in Ham's F-12 medium (Invitrogen) supplemented with 5% FBS NA, 5 µg/mL insulin, 1 µg/mL hydrocortisone (both from Sigma), and 1%penicillin/streptomycin (Biowest, Cat. # L0022). 4T1 cells were cultured in RPMI 1640 medium (Biowest, Cat #: L0500) supplemented with 10% FBS (Biowest, Cat. #: S1810) and 1%penicillin/streptomycin. All cells were tested for mycoplasma contamination routinely. Cells were maintained in a humidified, 5% CO2 atmosphere at 37°C.

*In vitro,* Short-Term starvation medium (STS) consists in a DMEM medium without glucose (DMEM no glucose, Life Technologies, Cat. #: 11966025) supplemented with 0.5 g/l glucose (Sigma-Aldrich, Cat. #: G8769) and 1% FBS. Control medium (CTR) consists in a DMEM no glucose medium supplemented with 1 g/l glucose and 10% FBS. For rescuing experiments with glucose/FBS, cells were cultured under CTR, STS, STS + 1g/l glucose-1%FBS, STS + 10%FBS-0,5g/l glucose and STS + 0,5g/l glucose-1%FBS and single FBS components at CTR concentration level (IGF1: 250ng/ml, EGF: 200ng/ml, Insulin: 200ng/ml) for a total of 48h.

### Reagents preparation

### WZB 117

Glucose transporter inhibitor IV, WZB117, was purchased from MERCK (Cat. #: 400036) and was dissolved in DMSO. Stock solutions of 70mg/ml were prepared for *in vivo* experiment and were stored at -80°C.

### Metformin

Metformin was purchased from Sigma-Aldrich (Cat. #: D150959) and was dissolved in sterile water to a final concentration of 1M (stock solution). Stock solutions were store at +4°C.

### 2-Deoxy-D-Glucose

2-Deoxy-D-Glucose was purchased from Sigma-Aldrich (Cat. #: D-6134) and was dissolved in sterile water to a final concentration of 2M (stock solution). Stock solutions were stored at +4°C.

### 8-Bromoadenosine 3',5'-cyclic mono-phosphates (8-Br-cAMP)

8-Br-cAMP was purchased from Cayman (Cat. #: 14431) and was dissolved in sterile water. Stock solutions of 25mg/ml were prepared and stored at -20°C.

### Alpelisib

Alpelisib was purchased from MedchemTronica (Cat. #: HY-15244) and was dissolved in DMSO. Stock solutions of 10mM were prepared for *in vitro* experiments and stored at -80°C.

### Rapamycin

Rapamycin was purchased from MedchemTronica (Cat. #: HY-10219) and was dissolved in DMSO. Stock solutions of 20mM and 20mg/ml were prepared for *in vitro* and *in vivo* experiments, respectively, and stored at -80°C.

### Pictilisib

Pictilisb was purchased from MedchemTronica (Cat. #: HY-50094) and was dissolved in DMSO. Stock solutions of 10mM and 200mg/ml were prepared for *in vitro* and *in vivo* experiments, respectively, and stored at -80°C.

### Ipatasertib

Ipatasertib was purchased from MedchemTronica (Cat. #: HY-15186) and was dissolved in DMSO. Stock solutions of 10mM and 200mg/ml were prepared for *in vitro* and *in vivo* experiments, respectively, and stored at -80°C.

### Erythrosin B exclusion assay

Cells were seeded in 12-well plates in their maintenance media for 24hours. The next days, cells were washed with PBS and grown in CTR/STS media for a total of 48h. After 24hours under CTR/STS conditions cells were treated with drugs or vehicle for the next 24 hours. In particular, for the experiment with PI3K-AKT-mTOR inhibitors, cells were treated with 10µM rapamycin, 10µM pictilisib, 20µM alpelisib and 20µM ipatasertib for a total of 24hours. To perform the erythrosin B exclusion assay, cells were suspended 1:1 with erythrosin B 0.1% in PBS (Sigma-Aldrich, Cat. #: 200964) and counted in a Burker chamber. The percentage of cell death was calculated as the ratio of erythrosine B-positive cells with the total number of cells.

### Mammosphere forming assay

For mammosphere formation assay *in vitro,* SUM159 and 4T1 cells were seeded in 12-well plates and grown in CTR/STS media for a total of 48h. At 24 hours, cells were treated with 5mM metformin or 4mM 2-Deoxy-D-Glucose. For rescuing experiments with PKA activator, cells were treated with 0,5mM 8-Br-cAMP, for a total of 24hours. To perform the mammosphere forming assay cells were digested into single cells using a 21G needle and then were plated in ultra-low attachment plates at a density of 500 or 1500 cells per well. Cells were cultured in a mammary epithelial basal medium (MEMB Cat. #: CC-3151) and methylcellulose (Sigma Cat. #: M0512) for 8/10 days. MEMB was previously supplemented with heparin (1U/ml), hydrocortisone (0,5 µg/ml), insulin (5µg/ml), 1% L-glutamine, 1% penicillin/streptomycin, B-27 (40µl/ml, Gibco Cat. #: 17504044), epidermal growth factor (EGF, 40ng/ml Biomol, Cat # BPS-90201-3) and fibroblast growth factor (FGF, 40ng/ml, Peprotech Cat. #: 100-18B). For *ex vivo* mammosphere formation assay, tumor masses were excised from the flank/ mammary fat pad of the mice and chopped in small parts with a scalpel and then digested enzymatically in DMEM medium supplemented with hyaluronidase (10mg/ml, Sigma Cat. #: H4272) and collagenase (2000U/ml, Sigma Cat. #: C2674) for 3 hours, 5%CO2 at 37°C. Cells obtained were filtered on cell strainer (100-70-40 µM) in order to achieve single cells and re-suspended in red blood cell lysing buffer hybrid-max (Sigma Cat. #: R7757) for 30 sec/1min. Finally, cells were plated in ultra-low attachment plates at a density of 1500 cells per well. To perform the serial sphere forming assay, mammospheres obtained were mechanically dissociated in single cells and re-plated to form secondary and tertiary spheres for 3 passages. 8/10 days after being plated, mammospheres with a diameter >60 µm were counted.

### CD44CD24 flow cytometer

CD44CD24 staining was performed both on SUM159 cells and on 4T1 tumour masses. 4T1 tumor tissues were enzymatically digested to obtain a single-cell suspension as previously described. Cells were harvested and washed in PBS 1% BSA and pellets were resuspended in blocking buffer (PBS 10% BSA) and incubated for 30 min at 4°C light protected. After a wash in PBS 1% BSA, cells were incubated with antibodies solution (200µl/1×10⁶ cells) for 45min at 4°C. In particular, 4T1 cells were stained with FITC-conjugated anti-murine CD24 antibody (Miltenyi Cat. #: 130-102-731, RRID: AB 2656573) and PE-conjugated anti-murine CD44 antibody (Miltenyi Cat. #: 130-102-606, RRID: AB 2658181), while SUM159 cells were stained with FITC-conjugated anti-human CD24 antibody (Miltenyi Cat. #: 130-112-844, RRID: AB2656554) and vioblue-conjugated anti-human CD44 antibody (Miltenyi Cat. #: 130-113-899, RRID: AB 2726390). SUM159 samples were analysed by flow cytometry with Attune NxT flow cytometer and data were processed by Kaluza analysis software (Beckman coulter, version 2.0). 4T1 samples were analysed by flow cytometry with FACSCalibur (BD) and data were processed by FlowJo software.

### Aldefluor assay

ALDEFLUOR kit (STEMCELL technologies, Cat. #: 01700) is used for the identification, evaluation and isolation of stem and progenitor cells expressing high levels of ALDH. SUM159 tumor tissues were dissociated enzymatically to obtain a single-cell suspension as previously described. Cells expressing high levels of ALDH become brightly fluorescent (ALDHbr) and were identified by flow cytometry with FACSCalibur (BD). Data were processed by FlowJo software.

### Immunohistological staining

Immunohistological analysis was performed to determine the expression of Caspase-3 protein in sample tissues of SUM159 tumor masses. Paraffin sections of 3 µm thickness were baked and prepared according to the procedure. Tumor masses slides were incubated overnight (4°C) with cleaved caspase-3 antibody (Asp175) (Euroclone Cat. #: BK9661S). Images of sections were taken by microscope (Upright BX 51 Full Manual) and the number of cells positive to Caspase-3 was calculated with ImageJ plugin (version 1.50i).

### Protein extraction and Western blot analysis

Cells were washed in ice-cold PBS and lysates were prepared in RIPA lysis buffer (50 mM Tris HCl pH 7.4, 150 mM NaCl, 1% NP-40, 0.25% deoxycholic acid, 1 mM EDTA) supplemented with protease and phosphatase inhibitors (protease inhibitor cocktail set III EDTA-free, Calbiochem, Cat. #: S39134; PhosStop, Roche).

Tumour tissues were collected and snap frozen in liquid nitrogen immediately after mice were sacrificed, and stored in -80 °C until use. For protein extraction, tumours were homogenized with Tissue lyser II (Qiagen) in RIPA buffer supplemented with protease and phosphatase inhibitors and then ultra-centrifuged (45000 rpm using MLA-130 Beckman rotor) for 1 hour. Protein concentrations were determined by BCA assay (Thermo Fisher Scientific, Cat. #: 23225). Proteins (30µg) were separated using pre-casted or home-made Acrylamid gels and transferred to 0.45µM nitrocellulose membranes or 0.2µM nitrocellulose membranes (depending on protein molecular weight). Proteins were then detected with the following antibodies: Vinculin (1:10000, Sigma-Aldrich, Cat. #: V9131, RRID: AB 477629), GLUT1 (1:10000, Cell Signaling, Cat. #: 12939, RRID: AB 2687899), CREB (1:1000, Cell Signaling, Cat. #: 4820S, RRID: AB 1903940), Phospho-CREB (1:1000, Cell Signaling, Cat. #: 9198S, RRID: AB 2561044), KLF5 (1:1000, Abcam, Cat. #: AB137676, RRID: AB 2744553), G9A/EHMT2 (1:1000, Euroclone, Cat. #: BK68851T) and histone H3dimethylK9 (1:1000, Abcam, Cat. #: AB1220, RRID: AB 449854). Next, membranes were washed with TBST (3 x 10 min) and incubated for 1h RT with anti-Mouse (1:3000, Cat. #: 170-6516) or anti-Rabbit (1:3000, Cat. #: 170-6515) secondary antibodies. Specific bindings were detected by a chemiluminescence system (Thermo Scientific). Bands intensity was quantified with Image Lab software (version 5.2.1).

### RNA extraction, RT-PCR and qRT-PCR

Total RNA was isolated using the miRNeasy Mini Kit (QIAGEN, #217004) according to the manufacturer's instructions. Next, 1 µg of purified RNA was retrotranscribed by using SuperScript Vilo cDNA synthesis kit (Invitrogen, #11754050). Resulting cDNA was analysed by real-time polymerase reaction (qRT-PCR) using Quant-Studio 12K flex Real Time PCR system (Thermo Fisher). Human target gene primers for NANOG (hs02387400_g1), OCT4 (hs00742896_s1), TBX3 (hs00195612_m1) and KLF2 (Hs00360439_g1) were used.

### CD44CD24 Cell sorting

SUM159 tumor masses were digested as previously described. Cells were washed in PBS 1% BSA and pellets were resuspended in blocking buffer (PBS 10% BSA) and incubated for 30 min at 4°C light protected. After a wash in PBS 1% BSA, cells were incubated with antibodies solution (200 µl/1×10⁶ cells) for 45min at 4°C. In particular, cells were stained with FITC-conjugated anti-human CD24 antibody (Miltenyi Cat. #: 130-112-844, RRID: AB 2656554) and vioblue-conjugated anti-human CD44 antibody (Miltenyi Cat. #: 130-113-899, RRID: AB 2726390). Finally, cells were washed with PBS 1% BSA and sorted with MoFlo Astrios Cell Sorter (Beckman Coulter).

### RNA sequencing

Libraries for RNA sequencing were prepared following the manufacturer protocols for transcriptome sequencing with the Illumina NextSeq 550DX sequencer (ILLUMINA).

Total RNA was isolated from cells, previously sorted, using the miRNeasy Mini Kit (QIAGEN, #217004), according to the manufacturer's instructions. RNA abundance was measured using Nanodrop and its integrity was assessed using Agilent Bioanalyzer 2100 with Nano RNA kit (RIN> 8). mRNA-seq indexed library preparation was performed starting from 500 ng of total RNA with the TruSeq stranded mRNA (Illumina). Indexed libraries were quality controlled on Agilent Bioanalyzer 2100 with High Sensitivity DNA kit, quantified with Qubit HS DNA, normalized and pooled to perform a multiplexed sequencing run. 1% PhiX control was added to the sequencing pool, as a positive run control. Sequencing was performed in PE (Paired-End) mode (2x75nt) on an Illumina NextSeq550Dx platform, generating on average 45 million PE reads per sample.

### RNA-seq Bioinformatics Analysis

Reads were aligned to the hg38 reference genome with STAR (doi: 10.1093/bioinformatics/bts635) with default settings and using the parameter quantMode GeneCounts in order to count the number reads per gene while mapping. Differential gene expression analysis between groups was performed with DESeq2 (doi: 10.1186/s13059-014-0550-8). Genes with |log2FC| > 2 and adjusted p value < 0.05 were considered as significantly deregulated.

Gene Set Enrichment Analysis (GSEA) for pathways of interest was performed on the fold change rank ordered gene list with the fgsea (doi: 10.1101/060012) package of Bioconductor. Volcano plots were generated with the R package ggplot2. Rendering of the fold changes on pathway graphs was achieved with the Pathview (doi: 10.1093/bioinformatics/btt285) Bioconductor package, which allows to download KEGG pathway graph data and render them with the mapped data.

### Mouse models

The animals were housed under specific pathogen-free conditions with 12 hours day/light cycles. All experiments were performed in accordance with the guidelines established in the Principles of Laboratory Animal Care (directive 86/609/EEC) and were approved by the Italian Ministry of Health. For xenograft experiments, 8-weeks old female NOD scid gamma (NSG, Charles River) were subcutaneously injected with 1,5×10⁶ SUM159 cells resuspended in 100 µl of PBS. For syngeneic model, 6-weeks old female Balbc/Ola Hsd mice (Envigo) were injected in the mammary fat pad with 2×10⁴ 4T1 cells resuspended in 20 µl of PBS. When tumors were palpable (approximately 7 days for SUM159 and 3 days for 4T1 after inoculation), mice were randomly divided in the different experimental groups. Body weights were recorded daily, and tumor volumes were measured twice a week by a digital caliper according to the following equation: tumor volume (mm3) = length x width x thickness x 0,5. At the end of the experiments, mice were euthanized by using CO2.

### Animal diets and treatments

Mice were fed ad libitum with irradiated VRFI (P) diet (Charles River) containing 3,89 kcal/g of gross energy. Our FMD is based on a nutritional screen that identified ingredients that allow nourishment during periods of low-calorie consumption⁹. The FMD diet consists of two different components designated as day 1 diet and days 2-4 diet. Day 1 diet consists of a mix of low-calorie broth powders, a vegetable powder, extra virgin olive oil and essential fatty acids; it contains 7.67 kJ/g (provided 50% of normal daily intake; 0.46 kJ/g protein, 2.2 kJ/g carbohydrate, 5.00 kJ/g fat). The day 2-3 diet is identical on all feeding days, consists of low-calorie broth powders and glycerol and contains 1.48 kJ/g (provided at 10% of normal daily intake; 0.01 kJ/g protein/fat,1.47 kJ/g carbohydrates). Mouse weight was monitored daily and during FMD cycle weight loss did not exceed 20%.

**Table 5. Energy density of FMD diets administered to mice.**

| | |
|---|---|
| FMD day 1 | 1.83 Kcal/g of food (0.11 Kcal/g proteins; 0.53 Kcal/g carbohydrates; 1.19 Kcal/g fatty acids); |
| FMD day 2-5 | 0.36 Kcal/g of food (0.0023 Kcal/g proteins; 0.35 Kcal/g carbohydrates; 0.0023 Kcal/g fatty acids). |

For experiments on tumor growth, mice were fed with standard diet (16% energy from proteins, 46% from carbohydrates, 38% from fat, total energy: 3.75 Kcal/g of food) or underwent FMD cycles (4 consecutive days per week). Before FMD cycle was repeated, mice completely recovered their original bodyweight.

For experiments performed to mimic the effect of FMD on CSCs or further reducing blood glucose levels, mice were daily treated with WZB117 (10mg/kg in PBS) via intraperitoneal injection, with metformin (150 mg/kg in PBS) via intraperitoneal injection, with 2-Deoxy-D-Glucose (500mg/kg in PBS) via intraperitoneal injection. For the experiment with PI3K-AKT-mTOR and CDK4/6 inhibitors, mice were treated with pictilisib for 5 consecutive days per week (100mg/kg in 10%DMSO, 40% PEG300 and 50% saline) by oral gavage, palbociclib every other day (62,5mg/kg in 5%DMSO, 40%PEG300 and 55% saline) by oral gavage, ipatasertib for 5 consecutive days per week (75mg/kg in 5%DMSO, 40%PEG300 and 55% saline) by oral gavage and rapamycin every other day (2mg/kg in 2%DMSO, 40%PEG300 and 58% saline) via intraperitoneal injection.

### Limiting dilution assay

For the limiting dilution assay, 8-weeks old female NOD scid gamma mice (NSG, Charles River) were subcutaneously injected with 1,5×10⁶ SUM159 cells resuspended in 100 µl of PBS. Mice were fed with standard diet or underwent FMD cycles (4 consecutive days per week) and were daily treated with 2DG (500mg/kg, via intraperitoneal injection) or vehicle. Body weights were recorded daily, and tumor volumes were measured twice a week by a digital caliper. After 5 weeks, donor mice were sacrificed and tumor masses were excised and digested, as previously described. Tumor cells derived from donor mice were re-injected at different dilution (100.000, 10.000, 1000 cells) in recipient 8-weeks old female NOD scid gamma mice. Recipient mice were always fed with standard diet and any drug wasn't administered; curves were performed considering the percentage of tumor palpability. Tumor initiating cell frequency was calculated with ELDA software.

### Survival analyses of TNBC patients

To evaluate the impact of blood glucose levels on overall survival (OS), advanced TNBC patients' data treated at "Fondazione IRCCS Istituto Nazionale dei Tumori" were retrospectively collected. Eligibility criteria were: 1) pathologically or cytologically confirmed diagnosis of clinical stage IV TNBC; 2) patients treated with at least one cycle of first-line, platinum-based doublet chemotherapy between October 2006 and January 2020; 3) available data regarding baseline (i.e. within 30 days from treatment initiation) plasma glucose concentration; 4) available information about patients' outcome. Platinum-based chemotherapy consisted of carboplatin-paclitaxel or carboplatin-gemcitabine combination. Carboplatin-paclitaxel doublet consisted of carboplatin at an Area Under the Curve (AUC) of 2 combined with paclitaxel 80 mg/mq, both administered intravenously on days 1 and 8 of every-three-week cycles. Carboplatin-gemcitabine consisted of carboplatin at an AUC of 2 combined with gemcitabine 800 mg/mq, both administered intravenously on days 1 and 8 of every-three-week cycles. OS was defined as the time between chemotherapy start and death from any cause. Patient data were collected according to the ethical principles for medical research involving human subjects adopted in the Declaration of Helsinki. Patients alive at the time of data collection and/or analyses signed an informed consent for the use of their personal data for research purposes.

### Statistical analysis

GraphPad Prism 8 was used for the analysis of the data and graphic representations. Comparisons between two groups were performed with two-tailed unpaired Student's t test. Comparison among more than two groups were performed with ANOVA analysis followed by Tukey's test. Comparison of survival curves were performed with Log-rank (Mantel-Cox) test. P values ≤ 0.05 were considered significant.

Survival curves of patients with TNBC were estimated by Kaplan Meier method and compared with the log-rank (Mantel-Cox) test. Patients who had not undergone death at the time of data cut-off and analysis were censored at their last disease evaluation. A threshold of significance of 0.05 was set for all statistical evaluations. Survival analyses of patient data were performed using the software R (version 4.0.2 (2020-06-22)).

### EXAMPLES

Note that only the AKT inhibitor Ipatasertib is part of the present invention.

### EXAMPLE 1: Fasting/FMD reduces human TNBC stem cells by lowering glucose levels.

In the *in vitro* human TNBC SUM159 model, fasting/FMD (low-serum, low-glucose conditions, referred to as Short-Term Starvation, STS) decreased mammosphere generation and volume, thus indicating an impact on TNBC self-renewal capacity (Fig. 5a). STS reduced also the proportion of CD44^{high}CD24^{low} cells, which are enriched for TNBC CSCs, compared to CTR conditions (Fig. 5b).

Consistent with *in vitro* results, cycles of FMD greatly reduces tumor progression and tumor size, associated with increased expression of Caspase3 (Fig. 5c, d). Moreover, when compared to ad libitum (AL) diet, FMD cycles decreased the number of *ex vivo* primary mammospheres, reducing their serial propagation, and the percentage of cells expressing aldehyde dehydrogenase 1 (ALDH1), an enzyme highly expressed in TNBC and associated with CSCs properties. (Fig. 1a, b). To further assess the effect of FMD on CSCs, a limiting dilution assay injecting tumor cells derived from *in vivo* xenografts in recipient mice, was performed at different cell dilutions. All recipient mice were fed an AL diet, regardless of the diet to which the donor mice had been subjected (AL or FMD). Results show that FMD decreases the frequency of cells able to promote TNBC, increasing cancer progression free survival, compared to AL (Fig. 5e).

The authors of the invention investigated whether the lowering glucose may be responsible for the sensitization of CSCs by fasting/FMD, and found that the STS-mediated sensitization is almost entirely rescued by the addition of physiological levels (1g/l) of glucose, while fetal bovine serum (FBS) or its main components (IGF1, EGF and insulin) supplementation did not have an effect on mammospheres growth (Fig. 5f). A similar experiment was also performed *in vivo,* by adding 3% of glucose to the drinking water of mice fed the FMD cycles, based on the normal concentration of sugar assumed daily by mice through the standard diet. Glucose supplementation partially reversed the FMD effects on tumor progression and completely rescued the FMD-dependent reduction of mammospheres, in agreement with the *in vitro* results (Fig. 1c, d, Fig. 5g).

To confirm the impact of glucose restriction on CSCs, the effect of WZB117, a specific inhibitor of the glucose transporter GLUT1(mostly expressed in TNBC) was evaluated. Cyclic FMD was more effective than WZB117 in halting tumor progression, in mice fed a standard diet, but the drug did not potentiate the anti-tumor activity of FMD, in agreement with the effect of FMD alone in decreasing the expression of GLUT1 (Fig 1e, Fig. 6a, b). Furthermore, WZB117 mimicked the effect of FMD on reducing the number of spheres formation, confirming the hypothesis that lower glucose levels reduce CSCs survival (Fig. 1f). Then, it was investigated the effect of compounds that act downstream of glucose uptake, namely 2Deoxy D-Glucose (2DG), an inhibitor of the glycolysis enzyme hexokinase, or the antidiabetic compound metformin, which reduces blood glucose levels and results in the inhibition of mitochondrial oxidative phosphorylation (OXPHOS). Interestingly, 2DG potentiated the effect of FMD both in delaying tumor progression and in decreasing the number of mammospheres derived by tumor masses, even after a multiple serial propagation, while it did not have any effect under AL conditions (Fig. 1g, h, Fig. 6c, d). Furthermore, in the limiting dilution assay, 2DG potentiated the effect of FMD in increasing mouse survival compared to 2DG and FMD alone, preventing tumor formation for more than 150 days at the 1,000 cells dilution level (Fig. 1i). On the other hand, metformin reduced tumor growth and sphere formation when compared with the control (AL diet), but it did not show any additive or synergistic antitumor effect when combined with the FMD, thus indicating that TNBC CSCs could be poorly sensitive to metformin (Fig. 6e-g). Collectively, these data indicate that CSCs are sensitive to glucose deprivation mediated by FMD and that 2DG potentiates FMD toxicity against CSCs.

### EXAMPLE 2: Low baseline glycemia is associated with increased survival of patients with metastatic TNBC.

To confirm data in a syngeneic mouse TNBC model, the effect of fasting/FMD in 4T1 mouse transplants in immuno-competent BALB/c female mice was tested. Notably, four FMD cycles delayed tumor progression, reduced mammospheres number as well as the percentage of CD44^{high}CD24^{low} subpopulation compared to AL conditions, confirming the effect of FMD on TNBC stem cells, using a different cell line (Fig. 2a, Fig. 7a-c). Moreover, consistent with data in human TNBC, supplementing 1 g/l of glucose reversed the STS effect on 4T1 sphere formation (Fig. 2b). Inventors also evaluated the effect of 2DG in combination with FMD and show that 2DG potentiated the effect of FMD both in delaying tumor progression and in reducing CSCs, confirming the results obtained with the SUM159 model (Fig 2c, d, Fig. 7d). To determine whether the findings in mice may be relevant to TNBC progression in humans, the relevance of blood glucose levels was investigated on the survival of patients with advanced TNBC. For this purpose, the inventors evaluated a retrospective series of advanced BC patients treated at Fondazione IRCCS Istituto Nazionale dei Tumori between October 2006 and January 2020 (Fig. 8). Out of 418 patients, a homogeneous group of advanced TNBC patients treated with first-line platinum-based chemotherapy (i.e. carboplatin-paclitaxel or carboplatin-gemcitabine doublets) was selected (Table 6). Patients were considered as normoglycemic if they have baseline blood glucose levels below 100 mg/dl when initiating chemotherapy. As shown in Fig. 2e, hyperglycaemic patients had significantly lower overall survival when compared with normoglycemic patients. Similar results were found when 110 mg/dl was chosen as a glycaemic threshold for hyperglycaemia definition (Fig. 2f). **In** line with results of preclinical experiments, these data indicate that blood glucose levels might also be important for the survival and progression of TNBC in patients treated with standard-of-care therapy.

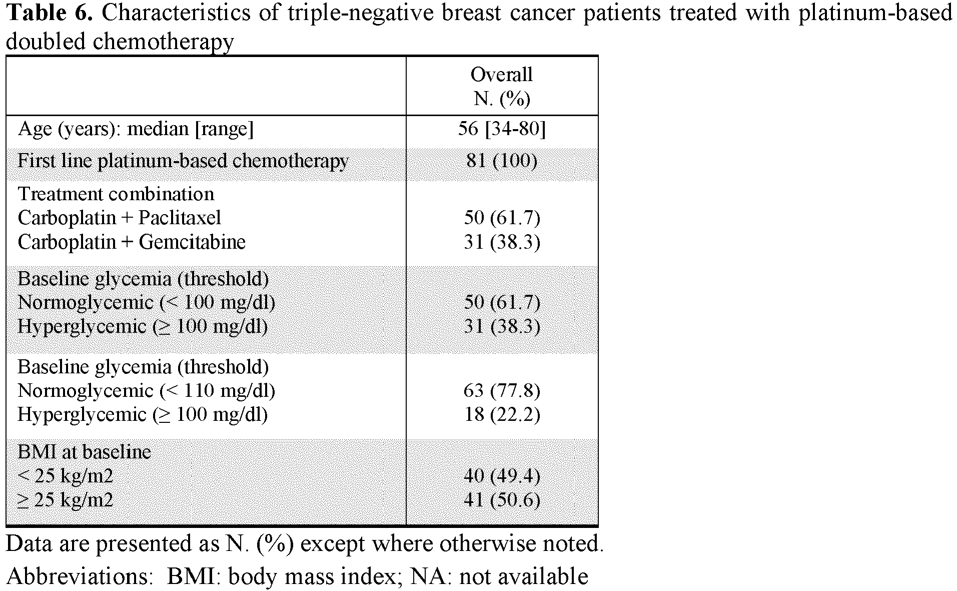

### EXAMPLE 3

### PKA activation reverses STS dependent mammosphere reduction.

Next, the mechanism through which glucose depletion sensitizes CSCs was investigated. It has been previously shown that prolonged fasting reduces the activity of protein kinase A (PKA) in different types of normal cells^{13,16}, an effect which is mediated in part by reduced glucose availability¹⁷. Interestingly, PKA inhibition results in the downregulation of human kruppel-like factor 5 (KLF5), a potential therapeutic target for TNBC¹⁸ through glycogen synthase kinase-3β (GSK3β) phosphorylation. KLF5 is reported to promote TNBC cell proliferation, survival, migration and invasion; moreover, it directly regulates the expression of some stemness associated genes, including Nanog and Oct4 (Shi P et al, Cell Discovery, 2017, 3, 17010). Therefore, KLF5 can be considered a potential target for TNBC stem cells. PKA inhibition is reported to downregulate KLF5; in fact, when PKA is downregulated, GSK3β phosphorylates KLF5 leading to its ubiquitination and degradation.

Thus, the involvement of PKA in FMD-dependent CSCs reduction was investigated. PKA activity was measured by examining the phosphorylation level of CREB, a classical downstream substrate of PKA, and KLF5 expression in SUM159 and 4T1 tumor masses. Notably, both of them were down-regulated in tumor masses in both models of mice treated with FMD, suggesting a PKA pathway inhibition (Fig. 3a). Moreover, RNA-seq analysis performed on SUM159 tumor masses, previously sorted for CD44CD24 human antibodies, revealed that FMD-induced inhibition of the PKA pathway selectively occurs in the CD44^{high}CD24^{low} staminal population but not in the CD44^{high}CD24^{high} differentiated cells, suggesting the selective involvement of PKA in affecting CSCs (Fig. 3b). In fact, the PKA activator 8-Bromoadenosine 3',5'-cyclic mono-phosphate (8-Br-cAMP) reversed fasting/STS-dependent spheres reduction both in the SUM159 and 4T1 models, confirming the role of PKA inhibition in stemness regulation (Fig. 3c, Fig 9a).

PKA also regulates the H3K9 methyltranferase G9A, which is implicated in the pathogenesis of several cancer types, including ovarian, lung, liver, breast and bladder cancers¹⁹⁻²². In particular, G9A-induced demethylation of H3K9 represses the expression of several tumor suppressor genes and adhesion molecules, promoting invasion and metastasis²³⁻²⁵. Both G9A and H3K9me2 levels were down-regulated by FMD in SUM159 and 4T1 tumor masses (Fig. 3d). Accordingly, FMD cycles, in SUM159 tumor masses, also reduced the mRNA of KLF5²⁶ and G9A²⁷ stemness-associated genes NANOG and OCT4, and KLF2 and TBX3, which are regulated by the transcriptional activity of OCT4 (Fig. 9b). Together, these data indicate that FMD-induced depletion of TNBC CSCs is at least mediated by glucose reduction and the inhibition of the PKA pathway.

### EXAMPLE 4

### FMD cycles cause TNBC regression and protect from the hyperglycemia induced by PI3K pathway inhibitors.

Even though TNBC progression is known to be CSCs dependent, differentiated cells contribution is also fundamental. In fact, fasting/FMD cycles alone were not effective in causing cancer free survival or long-term progression free survival, but only delayed tumor progression.

To identify potential druggable targets that may allow differentiated or CSCs to survive under fasting/FMD conditions, RNA-seq analysis on SUM159 tumor masses was performed. The analysis revealed that FMD cycles increase the expression of pro-apoptotic molecules, including BIM and ASK1, a critical cellular stress sensor frequently activated by ROS, whose production was previously shown to be increased by the FMD¹⁰. RNA-seq analysis also indicated that FMD cycles up-regulate PI3K-AKT and mTOR signalling (Fig. 4a, Fig. 10a), down-regulate CCNB-CDK1 while up-regulating CCND-CDK4/6 (Fig. 4a, Fig. 10a). Interestingly, the activation of survival factors by FMD cycles occurred only in differentiated cells, but not in CSCs (Fig. 10b).

Thus, it was investigated whether different PI3K/AKT and mTOR pathway inhibitors potentiate the effect of the FMD by blocking "starvation escape pathways (SEP)", necessary for survival. An *in* vitro experiment was performed to evaluate the effect of pictilisib (CDC-0941), a pan-PI3K inhibitor selective for all four isoforms of class I PI3Ks, which prevents the formation of phosphatidylinositol-triphosphate (PIP₃), key component of PI3K pathway, alpelisib (BYL-719), a low toxicity PI3Kα inhibitor, ipatasertib (GDC-0068), a highly selective pan-AKT inhibitor which binds to all three isoforms of AKT, and rapamycin (AY 22989), an allosteric mTOR inhibitor which binds to FK-binding protein 12 (FKBP12). All these inhibitors have anti-tumor activity in breast cancer models and also increase the toxicity of several drugs, reducing cancer cells resistance to treatments (O'Brien C et al., 2010; Tao JJ et al., 2014; Teo ZL et a., 2017). In particular, SUM159 TNBC cells were grown in CTR (1 g/L glucose; 10% FBS) and in STS (0,5 g/L glucose; 1% FBS) media for a total of 48h, and at 24h were treated with vehicle or pictilisib, alpelisib, ipatasertib or rapamycin. This screening identified pictilisib as the most efficacious in inducing cancer cell death under STS conditions, while seems to not have any effect under CTR conditions (Figure 10).

Results obtained *in vitro,* under fasting/STS conditions, show that the combination of Pictilisib, Ipatasertib and Rapamycin, selective inhibitors for PI3K, AKT and mTOR respectively, is very effective not only in inducing cancer cell death in SUM159 cells, but also in reducing the number of mammospheres (Fig. 10c, d). Results obtained in mouse xenografts of SUM159 indicate that weekly cycles of FMD improves significantly the anti-tumor effect of each inhibitor leading to a significative increase in mice survival (Fig. 4b, Fig. 11a). However, the most potent results were obtained when combining all three drugs with FMD cycles. In particular, treatment with the 3 drugs targeting PI3K/AKT and mTOR show a strong effect on progression free survival in mice fed with standard diet, but the addition of FMD cycles caused a major enhancement of the anti-tumor activity of the triple combination of PI3K pathway inhibitors, preventing tumor growth for more than 150 days in 85% of mice (Fig. 4c).

It is known that the hyperglycaemia caused by the mTOR inhibitor rapamycin sensitizes normal cells and mice to chemotherapy¹⁷. Hyperglycaemia caused by PI3K inhibitors is also well established to cause severe side effects in BC patients²⁸⁻³². Thus, it was tested whether the fasting/FMD cycles could also promote extended survival by preventing the side effects of hyperglycaemia. FMD cycles protected mice from the hyperglycaemia side effect of PI3K/AKT and mTOR inhibiting drugs (Fig. 4d); treatment with these drugs, caused a strong increase in blood glucose levels, leading to constant hyperglycaemia, in many cases resulting in death independently of tumor growth (Fig. 4c). FMD cycles restored glycemia to normal levels shortly after the drugs administration, thus allowing glucose levels to be normal during the great majority of the drug treatment (Fig. 4d). Moreover, based on RNA-seq results, the effect of the CDK4/6 inhibitor Palbociclib combined with Pictilisib was evaluated. Interestingly, CDK4/6 inhibitors were previously reported to sensitize PI3KCA mutated TNBCs to PI3K inhibitors³³. The obtained results revealed that combined treatment is very effective in delaying tumor progression and that FMD cycles further increases their anti-tumor activity and retards the acquisition of drugs resistance (Fig. 4e, Fig. 11b). In addition, the FMD-palbociclib-pictilisib triple treatment also reduced the formation of spheres compared to pharmacologic treatment alone after 8 weeks of treatment (Fig. 4e).

Finally, it was determined whether FMD cycles combined with targeted drugs can induce the reversal of late stage-tumor progression in human TNBC xenografts. SUM159-xenograft-bearing mice were injected with TNBC cells and either subjected to 4 cycles of FMD or fed with standard diet. 35 days post cells injection, when tumors were in an advanced growth stage, mice from the 2 groups were treated with the 3 inhibitors (Pictilisib, Ipatasertib, Rapamycin) plus FMD cycles.

The treatment of advanced stage tumors, either pre-treated with the FMD or fed a control diet, with FMD + the 3 drugs caused tumor regression within the first week of treatment. Notably, pre-treatment of mice with FMD cycles, prior to the start of the FMD + 3 drugs treatment, prevented acquisition of drugs resistance at a later stage, leading to complete tumor shrinkage and long-term progression free survival (Fig. 4f, Fig. 11c). In contrast, tumor masses of mice fed with standard diet, prior to the treatment with the FMD + 3 drugs, acquired resistance to the combination treatment (Fig. 4f). Together with the results described earlier in this study, this experiment suggests that the decrease of CSCs, caused by FMD cycles prior to the addition of the 3 drugs, is essential in preventing long-term acquisition of drug resistance and tumor growth.

### DISCUSSION

The failure of most anti-TNBC treatments in the advanced disease setting could be caused by the accumulation of a high number of treatment-resistant CSCs, which can repopulate the tumor masses after initial treatment-induced tumor debulking. Therefore, results herein obtained indicate that a treatment capable of depleting, or strongly reducing, TNBC CSCs, when tumors are in a less advanced stage, could strongly enhance the efficacy of subsequent treatments targeting both CSCs (such as the FMD) and more differentiated cancer cells (such as PI3K/AKT/mTORC1 inhibitors) in late-stage cancers.

In conclusion, FMD cycles allowed the use of RNA-seq data to identify druggable "starvation escape pathways" (SEPs) in differentiated cancer cells while reducing glucose levels, and consequently cancer stem cells number. However, these results also underline the "wild card" property of FMD cycles, which in addition to causing the activation of the druggable PI3K-AKT, mTOR, and CCND-CDK4/6 targets, reduced their ability to promote long-term hyperglycaemia and the side effects and deaths associated with it (Fig. 4c, d). This ability of FMD to protect from hyperglycaemia, a side effect which is limiting the use of PI3K and mTOR inhibiting drugs in the clinic, could provide benefits in the clinical setting in combination with a wide range of drugs well established to raise glucose levels, and including mTOR inhibitors rapamycin and everolimus, as well as PI3K inhibitors²⁸⁻³². Notably, high glucose levels could also promote high insulin levels, well known to promote cancer growth^{34.} Although in the *in vitro* experiments insulin does not appear to be driving cancer stem cells growth, this does not rule out an effect of insulin on differentiated cancer cells, since their growth is also affected by high glucose (Fig. 1c), in agreement with Yun J et al³⁵.

Notably, recent studies have shown that cyclic FMD is safe and feasible when combined with standard anti-tumor therapies in cancer patients^{14,36} and one clinical trial reported increased clinical and pathological efficacy of chemotherapy when administered to a group which included TNBC patients³⁶. The present invention clearly demonstrates that FMD cycles can reveal druggable starvation escape pathways in different tumor type and these pathways can be targeted by FMD in combination with inhibitors of the PI3K/AKT/mTORC1 and CDK4/6 axes in TNBC patients.

### REFERENCES

- 1.: Bianchi G, et al. Oncotarget. 2015 May 20;6(14):11806-19.
- 2.: Bulliard Y, et al., Immunol Cell Biol. 2014;92(6):475-480.
- 3.: Calabro L, et al., Lancet Oncol. 2013; 14:1104-11.
- 4.: Curti BD, et al. Cancer Res. 2013;73(24):7189-7198.
- 5.: Di Biase S, et al. Cancer Cell. 2016 Jul 11;30(1):136-146.
- 6.: Dougall WC, et al., Immunol Rev. 2017;276(1):112-120.
- 7.: El-Khoueiry AB et al. Lancet 389, 2492-2502 (2017).
- 8.: Esensten JH, et al., Immunity. 2016; 44(5):973-988.
- 9.: Foley EJ Cancer Res 13, 835-837 (1953).
- 10.: Fourcade J et al. J. Exp. Med 207, 2175-2186 (2010).
- 11.: Garon EB et al. N. Engl. J. Med 372, 2018-2028 (2015).
- 12.: Kim ST et al. Nat. Med 24, 1449-1458 (2018). [PubMed: 30013197]
- 13.: Larkin J, et al., N Engl J Med. (2015) 373:1270-1. doi: 10.1056/NEJMc1509660
- 14.: Lesokhin AM, et al., J. of Clinical Oncology, vol. 34, no. 23, pp. 2698-2704, 2016.
- 15.: Lee C, et al. Science Translational Medicine. 2012;4(124):124ra27.
- 16.: Lee C, Oncogene. 2011;30(30):3305-16. doi: 10.1038/onc.2011.91.
- 17.: Levine ME, et al. Cell Metabolism. 2014;19(3):407-17.
- 18.: Longo VD, Science 2003;299(5611):1342-6.
- 19.: Matsuzaki J et al. Proc. Natl Acad. Sci. USA 107, 7875-7880 (2010).
- 20.: Montler R, et al.. Clin Transl Immunol. (2016) 5:e70. 10.1038/cti.2016.16
- 21.: Motzer RJ et al. J. Clin. Oncol 33, 1430-1437 (2015).
- 22.: Mueller DL, Jenkins MK, Schwartz RH. Annu Rev Immunol. 1989;7:445-480.
- 23.: Piconese S, Valzasina B, Colombo MP. J Exp Med. 2008;205(6):1505].
- 24.: Postow MA, et al. N Engl J Med. (2015) 372:2006-17. doi: 10.1056/NEJMoa1414428
- 25.: Raffaghello L, et al. PNAS 2008; 105(24):8215-20.
- 26.: Ribas A, Hamid O, Daud A, Hodi FS, et al. JAMA. 2016 Apr 19;315(15):1600-9.
- 27.: Ribas A & Wolchok JD Science 359, 1350-1355 (2018).
- 28.: Robert C, et al. N Engl J Med. 2015 Jan 22;372(4):320-30.
- 29.: Robert C, et al. N Engl J Med. 2015 Jun 25;372(26):2521-32.
- 30.: Rosenberg JE et al. Lancet 387, 1909-1920 (2016).
- 31.: Sade-Feldman M., et al., Nat Commun. 2017 Oct 26;8(1):1136.
- 32.: Sangro B, et al. JHepatol.2013;59:81-8.
- 33.: Sarnaik, A.A. et al. Clin. Cancer Res. 17, 896-906 (2011).
- 34.: Schadendorf D, et al. J Clin Oncol. (2015) 33:1889-94.
- 35.: Shim HS, Wei M, Brandhorst S, Longo VD. Cancer Res. 2015 Mar 15;75(6):1056-67
- 36.: Slovin SF, et al. AnnOncol.2013;24:1813-21.
- 37.: Smith HJ Br. J. Cancer 20, 831-837 (1966).
- 38.: Topalian SL et al. N. Engl. J. Med 366, 2443-2454 (2012).
- 39.: Ward-Kavanagh LK, Lin WW, S 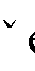 edy' JR, Ware CF. Immunity. 2016;44(5):1005-1019.
- 40.: Walunas TL et al. Immunity 1, 405-413 (1994).
- 41.: Weinberg AD, Rivera MM, Prell R, et al. J Immunol. 2000;164(4):2160-2169.
- 42.: Wikenheiser DJ et al., Front Immunol. 2016; 7:304.
- 43.: Wolchok JD, et al. N Engl J Med 2017; 377: 1345-1356.
- 44.: Wolchok, J.D. et al. Lancet Oncol. 11, 155-164 (2010).
- 45.: Yang JC, et al. JImmunother. 2007; 30:825-30.

## Claims

1. At least one reduced caloric intake cycle and at least one anticancer agent for use in the treatment of cancer wherein said at least one reduced caloric intake cycle provides at most 2100 Kcal per day and consists of a first and a second part, wherein the first part has a regular caloric intake reduced by 30% to 70% and the second part has a regular caloric intake reduced by 40 to 97%;
and wherein said at least one anticancer agent is the AKT inhibitor Ipatasertib.

2. The at least one reduced caloric intake cycle and at least one anticancer agent for use according to claim 1 further comprising at least one additional agent wherein said at least one additional agent is a PI3K inhibitor or a mTOR inhibitor or a CDK4/6 inhibitor.

3. The at least one reduced caloric intake cycle and at least one anticancer agent for use according to claim 2 wherein the PI3K inhibitor is pictilisib, alpelisib, paxalisib, rigosertib, dactolisib, MEN-1611, pilaralisib, VT-30, ART-001, CHF-6523, CUDC-908, HEC-68498, SF-1126, WXFL-10030390, YZJ-0673, AL-58805, AUM-302; the mTOR inhibitor is Rapamycin, everolimus, temsirolimus, onatasertib, bimiralisib, dactolisib , monepantel, sapanisertib, vistusertib, CC-115, CERC-006, detorsertib, FP-208; the CDK4/6 inhibitor is Palbociclib, abemaciclib, ribociclib, Trilaciclib, alvocidib, ebvaciclib, lerociclib, milciclib, SHR-6390, AT-7519, AZD-4573, BEY-1107, BPI-1178, CT-7001, FCN-437c, FIT-039.

4. The at least one reduced caloric intake cycle and at least one anticancer agent for use according to claims 2 or 3 wherein the AKT inhibitor is Ipatasertib, the PI3K inhibitor is Pictilisib or Alpelisib, the mTOR inhibitor is Rapamycin, the CDK4/6 inhibitor is Palbociclib.

5. The at least one reduced caloric intake and at least one anticancer agent for use according to anyone of previous claims, comprising administering a combination of:
- Ipatasertib and Rapamycin; or
- Ipatasertib and Pictilisib; or
- Ipatasertib and Alpelisib; or
- Ipatasertib and Rapamycin and Pictilisib; or
- Ipatasertib and Rapamycin and Alpelisib.

6. The at least one reduced caloric intake cycle and at least one anticancer agent for use according to any one of previous claims, wherein said first part and/or said second part lasts for a period of 24 to 190 hours, preferably said first part and/or or said second part lasts for a period of 24 to 120 hours, preferably said first part and/or or said second part lasts for approximately 120 hours.

7. The at least one reduced caloric intake cycle and at least one anticancer agent for use according to any one of previous claims, wherein the at least one reduced caloric intake cycle is repeated from 1 to 30 times after respective periods of from 5 to 60 days.

8. The at least one reduced caloric intake cycle and at least one anticancer agent for use according to any one of previous claims, comprising administering a further therapeutic intervention, preferably said further therapeutic intervention is selected from the group consisting of: surgery, radiotherapy and at least one further therapeutic agent, preferably said further therapeutic agent is a further AKT inhibitor, hexokinase inhibitor, PI3K inhibitor, mTOR inhibitor or CDK4/6 inhibitor, an immune checkpoint inhibitor, an immune response stimulator, a targeted anticancer agent, a DNA Damage Response inhibitor and/or a chemotherapeutic agent, preferably said further immune checkpoint inhibitor is selected from the group consisting of: PD1 inhibitors, PDL1 inhibitors, CTLA-4 inhibitors, TIGIT inhibitors, ICOS inhibitors, TIM3 inhibitors, IDO1 inhibitors; said immune response stimulator is selected from the group consisting of: OX40 activators, GITR modulators, 4-1BB agonists; said targeted anticancer agent is selected from the group consisting of: PI3K inhibitors, HDAC inhibitors, EGFR inhibitors, BRAF inhibitors, MAPK inhibitors, CDK inhibitors, ER stress activators; said DNA Damage Response inhibitor is selected from the group consisting of: PARP inhibitors, CHK1 inhibitors, ATR inhibitors, Wee1 inhibitors; said chemotherapeutic agent is selected from the group consisting of: Alkylating agents, Antimetabolites, Anti-microtubule agents, Topoisomerase inhibitors, Cytotoxic antibiotics.

9. The at least one reduced caloric intake cycle and at least one anticancer agent for use according to any one of previous claims, wherein said cancer is a solid or hematopoietic cancer, preferably the cancer is selected from the group consisting of: breast cancer, triple negative breast cancer, melanoma, lymphoma, lung cancer, non-small cell lung cancer (NSCLC), head and neck cancer, gastroesophageal cancer, bladder cancer and urothelial cancer, hepatocellular carcinoma and renal cell carcinoma.

10. The at least one reduced caloric intake cycle and at least one anticancer agent for use according to any one of previous claims, wherein said cancer is **characterized by** resistance or partial response to the treatment with at least one immunotherapeutic agent or anticancer agent.

## Patentansprüche

1. Mindestens ein Zyklus reduzierten Kalorienkonsums und mindestens ein Anti-Krebsmittel zur Verwendung bei der Behandlung von Krebs, wobei der mindestens eine Zyklus reduzierten Kalorienkonsums höchstens 2100 kcal pro Tag bereitstellt und aus einem ersten Teil und einem zweiten Teil besteht, wobei der erste Teil einen normalen Kalorienkonsum aufweist, der um 30 % bis 70 % reduziert ist, und der zweite Teil einen normalen Kalorienkonsum aufweist, der um 40 bis 97 % reduziert ist,
und wobei das mindestens eine Anti-Krebsmittel der AKT-Inhibitor Ipatasertib ist.

2. Mindestens ein Zyklus reduzierten Kalorienkonsums und mindestens ein Anti-Krebsmittel zur Verwendung nach Anspruch 1, ferner mindestens ein zusätzliches Mittel umfassend, wobei das mindestens eine zusätzliche Mittel ein PI3K-Inhibitor oder ein mTOR-Inhibitor oder ein CDK4/6-Inhibitor ist.

3. Mindestens ein Zyklus reduzierten Kalorienkonsums und mindestens ein Anti-Krebsmittel zur Verwendung nach Anspruch 2, wobei der PI3K-Inhibitor Pictisilib, Alpelisib, Paxalisib, Rigosertib, Dactolisib, MEN-1611, Pilaralisib, VT-30, ART-001, CHF-6523, CUDC-908, HEC-68498, SF-1126, WXFL-10030390, YZJ-0673, AL-58805, AUM-302 ist; der mTOR-Inhibitor Rapamycin, Everolimus, Temsirolimus, Onatasertib, Bimiralisib, Dactolisib, Monepantel, Sapanisertib, Vistusertib, CC-115, CERC-006, Detorsertib, FP-208 ist; der CDK4/6-Inhibitor Palbociclib, Abemaciclib, Ribociclib, Trilaciclib, Alvocidib, Ebvaciclib, Lerociclib, Milciclib, SHR-6390, AT-7519, AZD-4573, BEY-1107, BPI-1178, CT-7001, FCN-437c, FIT-039 ist.

4. Mindestens ein Zyklus reduzierten Kalorienkonsums und mindestens ein Anti-Krebsmittel zur Verwendung nach den Ansprüchen 2 oder 3, wobei der AKT-Inhibitor Ipatasertib ist, der PI3K-Inhibitor Pictilisib oder Alpelisib ist, der mTOR-Inhibitor Rapamycin ist, der CDK4/6-Inhibitor Palbociclib ist.

5. Mindestens ein reduzierter Kalorienkonsum und mindestens ein Anti-Krebsmittel zur Verwendung nach einem der vorhergehenden Ansprüche, umfassend Verabreichen einer Kombination von:
- Ipatasertib und Rapamycin; oder
- Ipatasertib und Pictilisib; oder
- Ipatasertib und Alpelisib; oder
- Ipatasertib und Rapamycin und Pictisilib; oder
- Ipatasertib und Rapamycin und Alpelisib..

6. Mindestens ein Zyklus reduzierten Kalorienkonsums und mindestens ein Anti-Krebsmittel zur Verwendung nach einem vorhergehender Ansprüchen, wobei der erste Teil und/oder der zweite Teil während einer Zeitspanne von 24 bis 190 Stunden andauert, bevorzugt der erste Teil und/oder der zweite Teil während einer Zeitspanne von 24 bis 120 Stunden andauert, der erste Teil und/oder der zweite Teil bevorzugt etwa 120 Stunden andauert.

7. Mindestens ein Zyklus reduzierten Kalorienkonsums und mindestens ein Anti-Krebsmittel zur Verwendung nach einem vorhergehender Ansprüchen, wobei der mindestens eine Zyklus reduzierten Kalorienkonsums 1 bis 30 mal nach jeweiligen Zeitspannen von 5 bis 60 Tagen wiederholt wird.

8. Mindestens ein Zyklus reduzierten Kalorienkonsums und mindestens ein Anti-Krebsmittel zur Verwendung nach einem vorhergehender Ansprüchen, umfassend Verabreichen eines weiteren therapeutischen Eingriffs, wobei der weitere therapeutische Eingriff bevorzugt aus der Gruppe ausgewählt wird bestehend aus: chirurgischem Eingriff, Radiotherapie und mindestens einem weiteren therapeutischen Mittel, wobei ferner das therapeutische Mittel bevorzugt ein weiterer AKT-Inhibitor, Hexokinase-Inhibitor, PI3K-Inhibitor, mTOR-Inhibitor oder CDK4/6-Inhibitor, ein Immun-Checkpoint-Inhibitor, ein Immunantwortanreger, ein Ziel-Anti-Krebsmittel, ein DNA-Schadenantwortinhibitor und/oder ein chemotherapeutisches Mittel ist, wobei bevorzugt der weitere Immun-Checkpoint-Inhibitor aus der Gruppe ausgewählt wird bestehend aus: PD1-Inhibitoren, PDL1-Inhibitoren, CTLA-4-Inhibitoren, TIGIT-Inhibitoren, ICOS-Inhibitoren, TIM3-Inhibitoren, IDOl-Inhibitoren; der Immunantwortanreger aus der Gruppe ausgewählt wird bestehend aus: OX40-Aktivatoren, GITR-Modulatoren, 4-1BB-Agonisten; das Ziel-Anti-Krebsmittelt aus der Gruppe ausgewählt wird bestehend aus: PI3K-Inhibitoren, HDAC-Inhibitoren, EGFR-Inhibitoren, BRAF-Inhibitoren, MAPK-Inhibitoren, CDK-Inhibitoren, ER-Stress-Aktivatoren; der DNA-Schadenantwortinhibitor aus der Gruppe ausgewählt wird bestehend aus: PARP-Inhibitoren, CHK1-Inhibitoren, ATR-Inhibitoren, Weel-Inhibitoren; das chemotherapeutische Mittel aus der Gruppe ausgewählt wird bestehend aus: Alkylierungsmitteln, Antimetaboliten, Anti-Mikrotubulusmitteln, Topoisomerase-Inhibitoren, cytotoxischen Antibiotika.

9. Mindestens ein Zyklus reduzierten Kalorienkonsums und mindestens ein Anti-Krebsmittel zur Verwendung nach einem vorhergehender Ansprüche, wobei der Krebs ein solider oder hämatopoetischer Krebs ist, der Krebs bevorzugt aus der Gruppe ausgewählt wird bestehend aus: Brustkrebs, triple-negatiem Brustkrebs, Melanom, Lymphom, Lungenkrebs, nicht-kleinzelligem Lungenkrebs (NSCLC), Kopf- und Halskrebs, Gastroösophagealkrebs, Blasenkrebs und urothelialem Krebs, hepatozellulärem Karzinom und Nierenzellkarzinom.

10. Mindestens ein Zyklus reduzierten Kalorienkonsums und mindestens ein Anti-Krebsmittel zur Verwendung nach einem vorhergehender Ansprüche, wobei der Krebs durch Resistenz oder teilweise Reaktion auf die Behandlung mit mindestens einem immuntherapeutischen Mittel oder Ani-Krebsmittel gekennzeichnet ist.

## Revendications

1. Au moins un cycle d'apport calorique réduit et au moins un agent anticancéreux destinés à être utilisés dans le traitement d'un cancer, dans lesquels ledit au moins un cycle d'apport calorique réduit fournit au plus 2100 Kcal par jour et est constitué d'une première et d'une seconde partie, dans lesquels la première partie a un apport calorique régulier réduit de 30 % à 70 % et la seconde partie a un apport calorique régulier réduit de 40 à 97 % ;
et dans lesquels ledit au moins un agent anticancéreux est l'inhibiteur d'AKT ipatasertib.

2. Au moins un cycle d'apport calorique réduit et au moins un agent anticancéreux destinés à être utilisés selon la revendication 1 comprenant en outre au moins un agent supplémentaire dans lesquels ledit au moins un agent supplémentaire est un inhibiteur de PI3K ou un inhibiteur de mTOR ou un inhibiteur de CDK4/6.

3. Au moins un cycle d'apport calorique réduit et au moins un agent anticancéreux destinés à être utilisés selon la revendication 2 dans lesquels l'inhibiteur de PI3K est le pictilisib, l'alpelisib, le paxalisib, le rigosertib, le dactolisib, MEN-1611, le pilaralisib, le VT-30, l'ART-001, CHF-6523, CUDC-908, HEC-68498, SF-1126, WXFL-10030390, YZJ-0673, AL-58805, AUM-302 ; l'inhibiteur de mTOR est la rapamycine, l'évérolimus, le temsirolimus, l'onatasertib, le bimiralisib, le dactolisib, le monépantel, le sapanisertib, le vistusertib, CC-115, CERC-006, le détorsertib, FP-208 ; l'inhibiteur de CDK4/6 est le palbociclib, l'abémaciclib, le ribociclib, le trilaciclib, l'alvocidib, l'ebvaciclib, le lérociclib, le milciclib, SHR-6390, AT-7519, AZD-4573, BEY-1107, BPI-1178, CT-7001, FCN-437c, FIT-039.

4. Au moins un cycle d'apport calorique réduit et au moins un agent anticancéreux destinés à être utilisés selon la revendication 2 ou 3 dans lesquels l'inhibiteur d'AKT est l'ipatasertib, l'inhibiteur de PI3K est le pictilisib ou l'alpélisib, l'inhibiteur de mTOR est la rapamycine, l'inhibiteur de CDK4/6 est le palbociclib.

5. Au moins un cycle d'apport calorique réduit et au moins un agent anticancéreux destinés à être utilisés selon l'une quelconque des revendications précédentes, comprenant l'administration d'une association de :
- ipatasertib et rapamycine ; ou
- ipatasertib et pictilisib ; ou
- ipatasertib et alpélisib ; ou
- ipatasertib et rapamycine et pictilisib ; ou
- ipatasertib et rapamycine et alpélisib.

6. Au moins un cycle d'apport calorique réduit et au moins un agent anticancéreux destinés à être utilisés selon l'une quelconque des revendications précédentes, dans lesquels ladite première partie et/ou ladite seconde partie dure une période de 24 à 190 heures, de préférence ladite première partie et/ou ladite seconde partie dure une période de 24 à 120 heures, de préférence ladite première partie et/ou ladite seconde partie dure environ 120 heures.

7. Au moins un cycle d'apport calorique réduit et au moins un agent anticancéreux destinés à être utilisés selon l'une quelconque des revendications précédentes, dans lesquels l'au moins un cycle d'apport calorique réduit est répété de 1 à 30 fois après des périodes respectives de 5 à 60 jours.

8. Au moins un cycle d'apport calorique réduit et au moins un agent anticancéreux destinés à être utilisés selon l'une quelconque des revendications précédentes, comprenant l'administration d'une intervention thérapeutique supplémentaire, de préférence ladite intervention thérapeutique supplémentaire est choisie dans le groupe constitué par : la chirurgie, la radiothérapie et au moins un agent thérapeutique supplémentaire, de préférence ledit agent thérapeutique supplémentaire est un inhibiteur d'AKT supplémentaire, un inhibiteur d'hexokinase, un inhibiteur de PI3K, un inhibiteur de mTOR ou un inhibiteur de CDK4/6, un inhibiteur de point de contrôle immunitaire, un stimulateur de réponse immunitaire, un agent anticancéreux ciblé, un inhibiteur de réponse aux dommages de l'ADN et/ou un agent chimiothérapeutique, de préférence ledit inhibiteur de point de contrôle immunitaire supplémentaire est choisi dans le groupe constitué par : les inhibiteurs de PD1, les inhibiteurs de PDL1, les inhibiteurs de CTLA-4, les inhibiteurs de TIGIT, les inhibiteurs d'ICOS, les inhibiteurs de TIM3, les inhibiteurs d'IDO1 ; ledit stimulateur de réponse immunitaire est choisi dans le groupe constitué par : les activateurs d'OX40, les modulateurs de GITR, les agonistes de 4-1BB ; ledit agent anticancéreux ciblé est choisi dans le groupe constitué par : les inhibiteurs de PI3K, les inhibiteurs de HDAC, les inhibiteurs d'EGFR, les inhibiteurs de BRAF, les inhibiteurs de MAPK, les inhibiteurs de CDK, les activateurs de stress du RE ; ledit inhibiteur de la réponse aux dommages de l'ADN est choisi dans le groupe constitué par : les inhibiteurs de PARP, les inhibiteurs de CHK1, les inhibiteurs d'ATR, les inhibiteurs de Weel ; ledit agent chimiothérapeutique est choisi dans le groupe constitué par : les agents alkylants, les antimétabolites, les agents antimicrotubules, les inhibiteurs de topoisomérase, les antibiotiques cytotoxiques.

9. Au moins un cycle d'apport calorique réduit et au moins un agent anticancéreux destinés à être utilisés selon l'une quelconque des revendications précédentes, dans lesquels ledit cancer est un cancer solide ou hématopoïétique, de préférence le cancer est choisi dans le groupe constitué par : le cancer du sein, le cancer du sein triple négatif, le mélanome, le lymphome, le cancer du poumon, le cancer du poumon non à petites cellules (CPNPC), le cancer de la tête et du cou, le cancer gastro-œsophagien, le cancer de la vessie et le cancer urothélial, le carcinome hépatocellulaire et le carcinome à cellules rénales.

10. Au moins un cycle d'apport calorique réduit et au moins un agent anticancéreux destinés à être utilisés selon l'une quelconque des revendications précédentes, dans lesquels ledit cancer est **caractérisé par** une résistance ou une réponse partielle au traitement avec au moins un agent immunothérapeutique ou un agent anticancéreux.
